# EUROPEAN PATENT SPECIFICATION

(11) **EP 1 523 336 B1**
(45) Date of publication and mention of the grant of the patent: **20.08.2008**
(21) Application number: 03765155.1
(22) Date of filing: 15.07.2003
(51) Int. Cl.: A61K 47/48, C07K 19/00

(54) **TARGETED AGENTS FOR NERVE REGENERATION**
GEZIELT EINGESETZTE AGENZIEN ZUR NERVENREGENERATION
AGENTS CIBLES DE REGENERATION DES NERFS

(30) Priority: 19.07.2002 GB 0216865
(43) Date of publication of application: 20.04.2005
(73) Proprietor: Syntaxin Limited, Abingdon Oxfordshire OX14 3YS (GB)
(72) Inventor: SHONE, Clifford Charles, Salisbury, Wiltshire SP4 0JG (GB); SUTTON, John, Mark, Salisbury, Wiltshire SP4 0JG (GB)
(74) Representative: MacLean, Martin Robert
(86) International application number: PCT/GB2003/003082
(87) International publication number: WO 2004/009126

(56) References cited:
- WO-A-00/28041
- WO-A-01/36588
- WO-A-01/58936
- WO-A-94/21300
- WO-A-99/08533
- US-A- 5 965 406
- CHADDOCK J A ET AL: "INHIBITION OF VESICULAR SECRETION IN BOTH NEURONAL AND NONNEURONAL CELLS BY A RETARGETED ENDOPEPTIDASE DERIVATIVE OF CLOSTRIDIUM BOTULINUM NEUROTOXIN TYPE A" , INFECTION AND IMMUNITY, AMERICAN SOCIETY FOR MICROBIOLOGY. WASHINGTON, US, VOL. 68, NR. 5, PAGE(S) 2587-2593 XP001010268 ISSN: 0019-9567 abstract page 2587, column 1, paragraph 2 page 2587, column 2, paragraph 1 page 2592, column 1, paragraph 1 page 2592, column 2

## Description

The present invention relates to delivery of agents to neuronal cells, especially agents that promote nerve regeneration, to constructs for delivering the agents, to associated use of the agents and constructs and to manufacture thereof.

There are presently few effective treatments for major disorders of the central nervous system. Such disorders include neurodegenerative diseases, stroke, epilepsy, brain tumours, infections and HIV encephalopathy, and sufferers of these diseases far outnumber the morbidity of cancer and heart disease. As our understanding of brain pharmacology increases and the underlying pathologies of diseases are elucidated, potential therapeutic strategies become apparent. All these treatments, however, face the formidable problem of efficient delivery of therapeutics to the various neuronal cell populations involved. Vectors which can effect efficient delivery to neuronal cells are thus required for a broad range of therapeutic substances, including drugs, enzymes, growth factors, therapeutic peptides and genes.

A major problem in the use of such therapies is the delivery of useful concentrations of the active agent to the site of trauma. Specific neuronal vectors could therefore play an important role in targeting such compounds to neuronal cells.

Suitable neuronal cell-specific targeting ligands are therefore required for a broad range of gene vectors to enable effective treatments for neuronal diseases to be developed.

WO 00/28041 describes a composition for the delivery of superoxide dismutase to neuronal cells, comprising a superoxide dismutase linked by a linker to a neuronal cell targeting component, which component comprises a first domain that binds to a neuronal cell and second domain that translocates the superoxide dismutase into the neuronal cell; following translocation, the linker is cleaved to release the superoxide dismutase.

WO 01/58936 describes a non-toxic polypeptide for delivery of a therapeutic agent to a neuronal cell, comprising a binding domain that binds to the neuronal cell, and a translocator domain that translocates the therapeutic agent into the neuronal cell; the translocation domain is not a H_{N} domain of a clostridial toxin and is not a fragment or derivative of a H_{N} domain of a clostridial toxin.

The clostridial neurotoxins are protein toxins produced by various species of the genus *Clostridium,* most importantly *C. tetani* and several strains of *C. botulinum.* There are at present eight different classes of the neurotoxins known: tetanus toxin and botulinum neurotoxin in its serotypes A, B, C₁, D, E, F and G, and they all share similar structures and modes of action. The clostridial neurotoxins are synthesized by the bacterium as a single polypeptide that is modified post-translationally to form two polypeptide chains joined together by a disulphide bond. The two chains are termed the heavy chain (H), and the light chain (LC). The clostridial neurotoxins are highly selective for neuronal cells, and bind with high affinity thereto.

The botulinum neurotoxins are a sub-family of clostridial neurotoxins whose primary site of action is the neuromuscular junction where they block the release of the transmitter acetylcholine. Tetanus toxin is structurally very similar to botulinum neurotoxins but its primary site of action is the central nervous system where it blocks the release of inhibitory neurotransmitters from central synapses (Renshaw cells).

The neuronal cell targeting of tetanus and botulinum neurotoxins is highly specific and is considered to be a receptor mediated event following which the toxins become internalised and subsequently traffic to the appropriate intracellular compartment where they effect their endopeptidase activity.

It is possible to provide functional definitions of the heavy chain domains within the clostridial neurotoxin molecules, as follows:-
clostridial neurotoxin heavy chain H_{c} domain:-
   - a portion of the heavy chain which is responsible for binding of the native holotoxin to cell surface receptor(s) involved in the intoxicating action of clostridial toxin prior to internalisation of the toxin into the cell.
clostridial neurotoxin heavy chain H_{N} domain:-
   - a portion of the heavy chain which enables translocation of that portion of the neurotoxin molecule such that a functional expression of light chain activity occurs within a target cell.
   - the domain responsible for translocation of the endopeptidase activity, following binding of neurotoxin to its specific cell surface receptor via the binding domain, into the target cell.
   - the domain responsible for formation of ion-permeable pores in lipid membranes under conditions of low pH.

Tetanus and the botulinum neurotoxins from most of the seven serotypes, together with their derived heavy chains, have been shown to bind a wide variety of neuronal cell types with high affinities in the nM range, e.g. botulinum type B neurotoxin (Evans et al. (1986) Eur. J. Biochem. 154, 409-416).

It is known to use H_{c} domains from botulinum toxins A and B to provide specific targeting of therapeutic agents to neuronal cells.

However, a problem that has been identified with these targeted constructs is that when the H_{c} domain is made recombinantly, the affinity and specificity for neuronal cells is significantly reduced, and this can be up to the point where there is no effective targeting of neuronal cells. To date, while attempts have been made to modify the H_{c} regions from A and B toxins to overcome this difficulty there has been no success.

Separately, it is known to use an adenoviral vector to deliver to a neuronal cell a gene that codes for C3 exoenzyme to promote central nervous system axon regeneration. This approach, however, has the disadvantage of the risk of viral proliferation in the patient receiving the treatment. As a result, it would be unlikely ever to receive approval for human use.

An object of the present invention is to provide agents that are targeted to neuronal cells and which can be used to deliver therapeutic agents thereto. An object of specific embodiments of the invention is to provide targeted delivery to neuronal cells of agents that can promote nerve regeneration. A further object is to overcome or at least improve upon the problems and disadvantages identified in the prior art.

Accordingly, a first aspect of the invention provides a conjugate according to Claim 1.

Thus according to the invention, an H_{c} portion from botulinum toxin C₁ is made recombinantly and used to provide targeting to neuronal cells of therapeutic agents. The H_{c} portion retains its binding affinity for neuronal cells, in contrast to the corresponding chains from A and B toxins.

Conjugates of the invention comprise a translation domain, for translocation of the therapeutic agent into a target cell. The neuronal cell targeting agent may be linked, e.g. covalently, using linkages which may include one or more spacer regions, to a translocation domain to effect transport of the therapeutic agent into the cytosol. Examples of translocation domains derived from clostridial neurotoxins are as follows:-

| | |
|---|---|
| Botulinum type A neurotoxin | - amino acid residues (449-871) |
| Botulinum type B neurotoxin | - amino acid residues (441-858) |
| Botulinum type C neurotoxin | - amino acid residues (442-866) |
| Botulinum type D neurotoxin | - amino acid residues (446-862) |
| Botulinum type E neurotoxin | - amino acid residues (423-845) |
| Botulinum type F neurotoxin | - amino acid residues (440-864) |
| Botulinum type G neurotoxin | - amino acid residues (442-863) |
| Tetanus neurotoxin | - amino acid residues (458-879) |

Other clostridial sources of translocation domains include - C. *butylicum,* and *C. argentinense,* and for the genetic basis of toxin production in *Clostridium botulinum* and *C. tetani,* see Henderson et al (1997) in The Clostridia: Molecular Biology and Pathogenesis, Academic press*.*

In addition to the above translocation domains derived from clostridial sources, other non-clostridial sources may be employed in a construct according to the present invention. These include, for example, diphtheria toxin [London, E. (1992) Biochem. Biophys. Acta., 1112, pp. 25-51], Pseudomonas exotoxin A [Prior et al (1992) Biochem., 31, pp.3555-3559], influenza virus haemagglutinin fusogenic peptides [Wagner et al (1992) PNAS, 89, pp.7934-7938*],* and amphiphilic peptides [Murata et al (1992) Biochem., 31, pp. 1986-1992*].*

In preferred embodiments of the invention, a translocation domain is selected from botulinum toxin C₁ translocation domain and functional fragments and derivatives thereof, and diphtheria toxin translocation domain and functional fragments and derivatives thereof.

In use, the domains of a conjugate according to the present invention are associated with each other. In one embodiment, two or more of the domains may be joined together either directly (eg. by a covalent linkage), or via a linker molecule. Conjugation techniques suitable for use in the present invention have been well documented:-
Chemistry of protein conjugation and cross-linking. Edited by Wong, S. S. 1993, CRC Press Inc., Florida; and
Bioconjugate techniques, Edited by Hermanson, G. T. 1996, Academic Press, London, UK.

Direct linkage of two or more domains is now described with reference to embodiments employing clostridial neurotoxins or fragments thereof and to the following nomenclature of clostridial neurotoxin domains, namely Domain B (contains the neuronal cell targeting domain), Domain T (contains the translocation domain) and Domain E (contains the therapeutic agent), although no limitation thereto is intended.

In one embodiment of the present invention, Domains E and T may be mixed together in equimolar quantities under reducing conditions and covalently coupled by repeated dialysis (eg. at 4° C, with agitation), into physiological salt solution in the absence of reducing agents. At this stage, in contrast to Example 6 of WO94/21300, the E-T complex is not blocked by iodoacetamide, therefore any remaining free -SH groups are retained. Domain B is then modified, for example, by derivatisation with the coupling agent SPDP followed by subsequent reduction. In this reaction, SPDP does not remain attached as a spacer molecule to Domain B, but simply increases the efficiency of this reduction reaction. Reduced Domain B and the E-T complex may then be mixed under non-reducing conditions (eg. at 4 °C) to form a disulphide-linked E-T-B construct.

In another embodiment, a coupled E-T complex may be prepared according to Example 6 of WO94/21300, including the addition of iodoacetamide to block free sulphydryl groups. However, the E-T complex is not further derivatised, and the remaining chemistry makes use of the free amino (-NH₂) groups on amino acid side chains (eg. lysine, and arginine amino acids).

Domain B may be derivatised using carbodiimide chemistry (eg. using EDAC) to activate carboxyl groups on amino acid side chains (eg. glutamate, and aspartate amino acids), and the E-T complex mixed with the derivatised Domain B to result in a covalently coupled (amide bond) E-T-B construct.

Suitable methodology for the creation of such a construct is, for example, as follows:-

Domain B was dialysed into MES buffer (0.1 M MES, 0.1 M sodium chloride, pH 5.0) to a final concentration of 0.5mg/ml. EDAC (1-ethyl-3-[3-dimethylaminopropyl] carbodiimide hydrochloride) was added to final concentrations of 0.2 mg/ml and reacted for 30 min at room temperature. Excess EDAC was removed by desalting over a MES buffer equilibrated PD-10 column (Pharmacia). The derivatised domain B was concentrated (to >2mg/ml) using Millipore Biomax 10 concentrators. The E-T complex (1 mg/ml) was mixed for 16 hours at 4 °C, and the E-T-B complex purified by size-exclusion chromatography over a Superose 12 HR10/30 column (Pharmacia) to remove unreacted Domain B (column buffer: 50mM sodium phosphate pH6.5 + 20mM NaCl).

As an alternative to direct covalent linkage of the various Domains of a conjugate according to the present invention, suitable linker molecules may be employed. The term linker molecule is used synonymously with spacer molecule. Spacer technology was readily available prior to the present application.

For example, one particular coupling agent (SPDP) is described in Example 6 of WO94/21300 (see lines 3-5 on page 16). In Example 6, SPDP is linked to an E-T complex, thereby providing an E-T complex including a linker molecule. This complex is then reacted with a Domain B, which becomes attached to the E-T complex via the linker molecule. In this method, SPDP results in a spacing region of approximately 6.8 Angstroms between different Domains of the conjugate of the present invention.

A variant of SPDP known as LC-SPDP is identical in all respects to SPDP but for an increased chain length. LC-SPDP may be used to covalently link two Domains of the conjugate of the present invention resulting in a 15.6 Angstrom spacing between these Domains.

Examples of spacer molecules include, but are not limited to:-

| | |
|---|---|
| (GGGGS)₂, elbow regions of Fab | - [see Anand et al. (1991) J. Biol. Chem. 266, 21874-9]; |
| (GGGGS)₃ | - [see Brinkmann et al. (1991) Proc. Natl. Acad. Sci. 88, 8616-20]; |
| the interdomain linker of cellulase | - [see Takkinen et al. (1991) Protein Eng, 4, 837-841]; |
| PPPIEGR | - [see Kim (1993) Protein Science, 2, 348-356]; |
| Collagen-like spacer | -[see Rock (1992) Protein Engineering, vol 5, No 6, pp583-591]; and |
| Trypsin-sensitive diphtheria toxin peptide | - [see O'Hare (1990) FEBS, vol 273, No 1,2, pp 200-204]. |

In a further embodiment of the present invention, a conjugate having the structure E-X-T-X-B, where "X" is a spacer molecule between each domain, may be prepared, for example, as follows:-

Domain E is derivatised with SPDP, but not subsequently reduced. This results

in an SPDP-derivatised Domain E. Domain T is similarly prepared, but subsequently reduced with 10mM dithiothreitol (DTT). The 10mM DTT present in the Domain T preparation, following elution from the QAE column (see Example 6 in WO94/21300), is removed by passage of Domain T through a sephadex G-25 column equilibrated in PBS. Domain T free of reducing agent is then mixed with the SPDP-derivatised Domain E, with agitation at 4 °C for 16 hours. E-T complex is isolated from free Domain E and from free Domain T by size-exclusion chromatography (Sephadex G-150). Whereafter, the same procedure can be followed as described in Example 6 of WO94/21300 for rederivatisation of the E-T complex with SPDP, and subsequent coupling thereof to the free sulphydryl on Domain B to form.

In a conjugate of the invention, the translocation domain (Domain T) is not limited, and could be any translocation domain. Translocation domains can frequently be identified by the property of being able to form measurable pores in lipid membranes at low pH (Shone et al. Eur J. Biochem. 167, 175-180).

The therapeutic agent could, similarly, be any suitable agent and reference to "therapeutic substance" or "therapeutic agent" is a reference to any substance, agent or mixture thereof, which, if delivered by or in the composition of the invention, is beneficial in the treatment of disease. Examples of these include drugs, growth factors, enzymes, and DNA packaged in various forms (e.g. modified viruses, cationic liposomes, and condensed DNA).

For promoting nerve regeneration, any Rho inhibitor can be used, especially a C3 exoenzyme. The C3 family of exoenzymes is defined as a family of proteins of 20-30kDa with a basic isolectric point usually greater than 9. The enzymes are ADP-ribosyltransferases, which modify small GTPases, usually of the Rho family, by the addition of ADP-ribose. This usually results in the inactivation of the GTPases.

Members of the C3 exoenzyme family described to date include at least 2 isoforms from *Clostridium botulinum,* an isoform from *C.limosum,* an isoform from *Bacillus cereus,* and 3 isoforms from *Staphylococcus aureus.* C3 isoforms from *C.acetobutylicum, Streptococcus pyogenes* and *Listeria monocytogenes* are disclosed for the first time herein and represent further embodiments of the invention.

Enzymes having C3 activity can be identified, for example, using the assay described in Example 1.

C3-like toxins can be further defined by the presence of motifs covering two elements of the active site such that all examples carry the consensus S S/T S/T Hyd X35-40 Q/E X E Hyd Hyd Hyd where Hyd represents any hydrophobic residue (usually I, L, V, G or A) and X represents any amino acid. The motif usually has an aromatic residue in one of the two preceding positions. The S S/T S/T motif is a key determinant of substrate specificity. Hence, a C3 enzyme used in the present invention is preferably one that includes these motifs.

In use, advantages of constructs of the invention include one or more of:-
targeted to the neuron, therefore no cytotoxic effect on non-neuronal cells,
the enzymic action of C3, in which one C3 molecule can inactivate many target (Rho) molecules, provides efficient inhibition which is more effective than conventional (drug-like) inhibitors which act by binding to their target in a one-to-one ratio,
much smaller molecular size compared to virus vectors, therefore better penetration of tissues *in vivo,*
better safety profile compared to viral delivery vectors with no risk of uncontrolled replication *in vivo,*
delivery of functional protein to cells with no delay in action which contrasts to DNA delivery in which translation of the DNA into the protein has to occur before an effect,
constructs of the invention are less immunogenic than viral or DNA constructs,
constructs prepared using C3 from *Staphylococcus* species have the advantage that they are not inhibited by the naturally occurring intracellular protein RalA,
botulinum toxin-targeted C3 rapidly bind to neurons due to their high affinity receptor interaction resulting in efficient action and reduced immune responses, and
possible to specifically target Rho isoforms other than the cytoplasmic RhoA; for example, targeting of the endosomal RhoB has significant therapeutic potential.

A modified clostridial heavy chain for the conjugate of the invention is Botulinum suitably produced by combining the binding domain (H_{C} domain) of a Botulinum C1 toxin with a non-clostridial translocation domain. Thus, for example, a modified clostridial heavy chain fragment may be constructed from the translocation domain of diphtheria toxin (residues 194-386) fused to the H_{c} domain of a botulinum C1 toxin.

In another embodiment of the invention, the modified clostridial heavy chain is produced by combining the H_{c} domain of a Botulinum C1 toxin with a membrane disrupting peptide which functions as a translocation domain, suitably a viral peptide. Thus, for example, a modified clostridial heavy chain fragment may be constructed by combining the H_{c} domain of a botulinum C1 toxin with a peptide based on influenza virus haemagglutinin HA2 (residues 1-23).

In another embodiment of the invention, the modified clostridial heavy chain fragment is fused to a linker peptide via the N-terminus of the translocation domain, to which linker peptide a polypeptide payload may be attached. An example of such a linker peptide is the sequence CGLVPAGSGP (SEQ ID NO:23) which contains the thrombin protease cleavage site and a cysteine residue for disulphide bridge formation. Such a peptide linker allows production of a recombinant fusion protein comprising a polypeptide therapeutic molecule fused by the linker peptide to the N-terminus of the modified clostridial heavy chain fragment. The latter single chain fusion protein may then be treated with thrombin to give a dichain protein in which the polypeptide therapeutic is linked to the translocation domain of the modified clostridial heavy chain fragment by a disulphide link between a free cysteine on the translocation domain and a cysteine residue of the linker peptide. In another example of a linker peptide in which the translocation domain does not contain a free cysteine residue near its C-terminus, such as is the case when the translocation domain is a fusogenic peptide, the linker peptide contains both cysteine residues required for the disulphide bridge. An example of the latter linker peptide is the amino acid sequence: CGLVPAGSGPSAGSSAC (SEQ ID NO:24).

In yet another embodiment of the invention, the modified clostridial heavy chain is linked directly or indirectly to DNA such that the construct is capable of delivering the DNA to neuronal cells. Such constructs have gene therapy applications and be used to switch on, or off, selected genes with the cell. The DNA may be contained within a liposome or be condensed via a peptide or protein. The modified clostridial heavy chain may be chemically linked to the protein that effects the DNA condensation by chemical coupling agents. Alternatively, the modified clostridial heavy chain may be produced as a fusion protein, by recombinant technology, with a peptide that can effect the condensation of DNA.

In yet another embodiment of the invention, the modified clostridial heavy chain fragment may be linked to a recombinant virus such that the modified virus has an altered tropism and is capable of transducing cells. Such a construct is of use to correct genetic defects within neuronal cells by switching on, or off, selected genes. The modified clostridial heavy chain fragment may be linked directly to the surface of the virus using chemical cross-linking agents. Alternatively the modified clostridial heavy chain fragment may be linked to the recombinant virus via an antibody which specifically bind to the virus. In this instance the modified clostridial heavy chain fragment is chemically coupled to a polyclonal or monoclonal antibody which specifically recognizes a marker on the surface of the virus. A similar modified clostridial heavy chain fragment-antibody fusion protein could be produced by recombinant technology in which the antibody component is a recombinant single chain, antibody.

In yet another embodiment of the invention, the modified clostridial heavy chain fragment is linked to a drug release system such as a microparticle constructed from a suitable polymer, e.g. poly (lactide-co-glycolide), polyhydroxylalkonate, collagen, poly(divinyl-ether-comaleic anhydride, poly (styrene-co-maleic anhydride) or other polymer useful in such microparticles. The modified clostridial heavy chain fragment may be linked to the drug release system by covalent chemical coupling, or electrostatic or hydrophobic forces. The modified clostridial heavy chain fragment may also be encapsulated within the release vehicle together with the therapeutic payload provided that a portion of the modified clostridial binding domain is exposed at the surface. Alternatively, the modified clostridial heavy chain fragment may be linked, at either the N- or C-terminal end, to a peptide or protein to facilitate coupling of the fragment to the drug release system.

Other strategies are known by which modified clostridial heavy chain fragments can be linked to range of therapeutic substances using a variety of established chemical cross-linking techniques, and a variety of fusion proteins can be produced containing a modified clostridial binding fragment and another polypeptide. Using these techniques a variety of substances can be targeted to neuronal cells using the modified clostridial heavy chain fragments. Examples of possible uses of the modified clostridial heavy chain fragments as neuronal delivery vectors are given in more detail below. conjugate of the invention may be introduced into either neuronal or non-neuronal tissue using methods known in the art. By subsequent specific binding to neuronal cell tissue, the targeted conjugate exerts its therapeutic effects. Ideally, the conjugate is injected near a site requiring therapeutic intervention.

The conjugate of the invention may be produced as a suspension, emulsion, solution or as a freeze dried powder depending on the application and properties of the therapeutic substance. The conjugate of the invention may be resuspended or diluted in a variety of pharmaceutically acceptable liquids depending on the application.

"Clostridial neurotoxin" means either tetanus neurotoxin or one of the seven botulinum neurotoxins, the latter being designated as serotypes A, B C₁, D, E, F or G.

"Modified clostridial heavy chain fragment" means a polypeptide fragment which binds to neuronal cell receptors in similar manner to a corresponding heavy chain derived from botulinum or tetanus toxins but differs in its amino acid sequence and properties compared to the corresponding fragment derived from botulinum or tetanus toxin.

"Bind" in relation to the botulinum and tetanus heavy chain fragments, means the specific interaction between the clostridial fragment and one or more cell surface receptors or markers which results in localization of the binding fragment on the cell surface. In the case of the clostridial neurotoxins, the property of a fragment being able to 'bind' like a fragment of a given serotype can be demonstrated by competition between the ligand and the native toxin for its neuronal cell receptor.

"High affinity binding specific to neuronal cell corresponding to that of a clostridial neurotoxin" refers to the ability of a ligand to bind strongly to cell surface receptors of neuronal cells that are involved in specific binding of a given neurotoxin. The capacity of a given ligand to bind strongly to these cell surface receptors may be assessed using conventional competitive binding assays. In such assays radiolabelled clostridial neurotoxin is contacted with neuronal cells in the presence of various concentrations of non-radiolabelled ligands. The ligand mixture is incubated with the cells, at low temperature (0-3°C) to prevent ligand internalization, during which competition between the radiolabelled clostridial neurotoxin and non-labelled ligand may occur. In such assays when the unlabelled ligand used is the same as that of the labelled neurotoxin, the radiolabelled clostridial neurotoxin will be displaced from the neuronal cell receptors as the concentration of non-labelled neurotoxin is increased. The competition curve obtained in this case will therefore be representative of the behaviour of a ligand which shows "high affinity binding specificity to neuronal cells corresponding to that of a clostridial neurotoxin", as used herein.

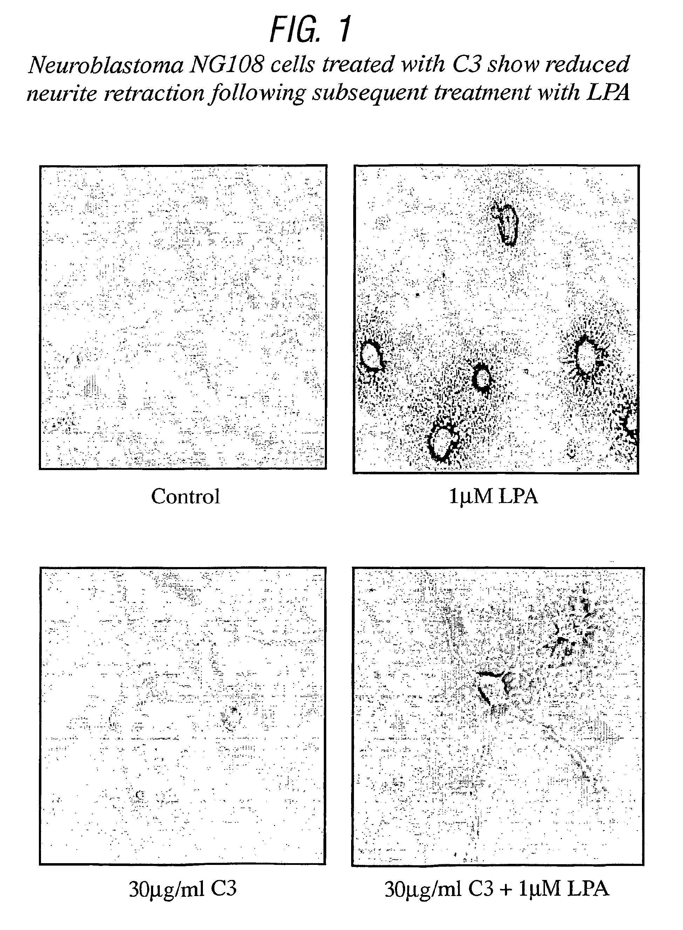

"Translocation domain" means a domain or fragment of a protein which effects transport of itself and/or other proteins and substances across a membrane or lipid bilayer. The latter membrane may be that of an endosome where translocation will occur during the process of receptor-mediated endocytosis. Translocation domains can frequently be identified by the property of being able to form measurable pores in lipid membranes at low pH (Shone et al. Eur J. Biochem. 167, 175-180). Examples of translocation domains are set out in more detail below in Figure 1. In the application, translocation domains are frequently referred to as "H_{N} domains".

"Translocation" in relation to translocation domain, means the internalization events which occur after binding to the cell surface. These events lead to the transport of substances into the cytosol of neuronal cells.

"Therapeutic substances" or "agents" mean any substance, agent or mixture thereof, which, if delivered by the modified clostridial binding fragment, would be beneficial to the treatment of neuronal diseases. Examples of these include drugs, growth factors, enzymes, and DNA packaged in various forms (e.g. modified viruses, cationic liposomes, and condensed DNA).

"C3-like activity" means that an enzyme expresses an ADP-ribosyl transferase activity which is specific for small cellular GTPases of the Rho family. In this activity, the C3-like enzyme catalyses the transfer of an ADP-ribose moiety from NAD to the Rho GTPase which usually results in an loss of function of the GTPase.

Also provided in the present description are methods of manufacture of the polypeptides of the invention by expressing in a host cell a nucleic acid encoding the polypeptide, and the use of a polypeptide or a composition according to the invention in the treatment of a disease state associated with neuronal cells.

The invention is now illustrated in the following specific embodiments and accompanied by drawings in which:-
Fig. 1 shows protection against neurite reaction in LPA-treated neuroblastoma cells by C3 enzyme; and
Fig. 2 shows protective or neurostimulatory effect of C3 on LPA-treated neurons.

### Examples:

### 1. Measurement of exoenzyme C3 activity within an enzyme

To carry out an assay for the exoenzyme C3, an assay mix was prepared containing the following components:-
- 10µl of Assay Buffer (0.05M Hepes pH 7.2 containing MgCl₂ to a final concentration of 2mM)
- 20µl of recombinant Rho A solution in Assay Buffer (to give 0.5Ng per 100µl assay)
- 20µl of [³²P] NAD⁺ solution in Assay Buffer (to give 1 µCi per 100µl assay). The NAD being labelled in the alpha phosphate position.

This mixture was incubated at 37°C for 5 min then 50µl of C3 enzyme solution (prewarmed to 37°C) added to start the enzymic reaction.

After incubation for various times at 37°C (typically between 15-120min), 100µl of BSA (2mg/ml) was added to each assay mix followed by 0.5ml of 24% trichloroacetic acid solution (TCA) to stop the reaction. The resulting precipitate was centrifuged at high speed on a microfuge for 2 mins, the supernatant fluid decanted and then the precipitate washed with a further 1 ml of 12%TCA solution. The precipitate was resuspended in 1ml of PBS, by passaging several times through a pipette tip, and then 5ml scintillation fluid added, mixed and counted in scintillation counter.

The presence of exoenzyme C3, or an enzyme with exoenzyme C3-like activity, was confirmed by the presence of significantly higher radioactivity in the pellet compared to control incubations containing no C3 enzyme.

### 2. Cloning and expression of C3 genes.

Standard molecular biology protocols were used for all genetic manipulations (Sambrook et al 1989, Molecular cloning; A laboratory manual. Second Edition, Cold Spring Harbor Laboratory Press, New York.). C3 genes were amplified by PCR to generate suitable restriction sites for cloning. In some cases synthetic genes were prepared with codon usage optimised for expression in *E.coli.* The restriction sites *Bam*HI (5') and *Bg*/II (3') were used for most cloning operations with reading frames designed to start with the first base of the restriction site. Constructs were sequenced to confirm the presence of the correct sequence. An expression vector containing the malE gene from the pMAL-C2x (NEB) subcloned as an *Apa*I*-Hind*III fragment into the cloning vector pBC (Strategene) digested *Apa*I*-Hind*III was prepared. The C3 genes were cloned into the *Bam*HI site as *Bam*HI-*Bgl*II fragments. Alternatively the C3 genes were cloned into either an expression vector carrying a T7 polymerase promoter site (e.g. pET28, pET30 or derivatives (Novagen Inc, Madison, WI)) or as a fusion with maltose binding protein (e.g. pMALc2x (NEB)) as a suitable fragment. Clones with confirmed sequences were used to transform expression hosts. Strain TB1 was used for most expression with alternative hosts used as required (For T7 polymerase vectors *E.coli* BL21 (DE3) (Studier and Moffatt 1986 Journal of Molecular Biology 189:113-130) JM109 (DE3) or equivalent strains with a DE3 lysogen. For pMalc2x JM109, BL21, TG1, TB1 or other suitable expression strains).

In addition to the expression of C3 proteins as standard fusion proteins an additional approach was used to generate fusions for direct assembly into targeting vectors. C3 fragments were cloned into a BamHI site introduced at the 5' end of a neuronal cell targeting moiety such as a hybrid diphtheria translocation domain-clostridial neurotoxin binding domain fusion protein

Expression cultures of TB1 pBCmalE C3 were grown in Terrific Broth containing 35µg/ml chloramphenicol and 0.5% (w/v) glucose to an OD₆₀₀ of 2.0 at 30°C and cultures were induced with 500µM IPTG and grown at 25°C for 2 hours. Other expression systems used similar conditions except that the antibiotic was changed to either kanamycin or ampicillin. Cells were lysed by either sonication in column buffer (20mM Hepes 100mM NaCl pH 6.8) or suitable detergent treatment (e.g. Bugbuster reagent; Novagen) and cell debris pelleted by centrifugation. Supernatant proteins were loaded onto a SP-sepharose Fast Flow column equilibrated in column buffer and proteins eluted with a gradient of 0-1 M NaCl. MBP-C3 fusions eluted at 200mM NaCl. Fusion protein was cleaved with Factor Xa in 20mM Hepes 200mM NaCl pH6.8 (as eluted) to separate the C3 domain from its MBP fusion partner. The protein was diluted to give a final buffer concentration of 20mM Hepes 100mM NaCl pH 6.8 and loaded onto the SP-Sepharose column under identical conditions to those used initially. C3 protein,essentially free of contaminating proteins, was eluted at -400 mM NaCl. Protein was stored at -70°C until required.

The C3stau2 isoform can be purified using a similar method. Cells are lysed in 20mM MES 50mM NaCl pH5.8 and the soluble material loaded on to an SP-sepharose column. The protein elutes at around 150mM NaCl. The protein is dialysed against 20mM HEPES 50mM NaCl pH 7.3 and cleaved with Factor Xa. The protein is passed through a second SP-sepharose column and elutes at approximately 250mM NaCl. Protein is stable at -70°C.

His-tag fusions were loaded onto a metal chelate column charged with Cu²⁺ (Amersham-Pharmacia Biotech, Uppsala, Sweden). After loading proteins on the column and washing, proteins were eluted using imidazole. All buffers were used as specified by manufacturers. Where appropriate removal of the purification tag was carried out according to manufacturers instructions.

MBP fusions could also be purified on amylose resin columns as described by the manufacturer (NEB) following growth in Terrific Broth containing 100 µg/ml ampicillin and lysis as described above.

### 3. Expression of heavy chain fragments.

Standard molecular biology protocols were used for all genetic manipulations (Sambrook et al 1989, Molecular cloning; A laboratory manual. Second Edition, Cold Spring Harbor Laboratory Press, New York.) Clostridial neurotoxin binding domains (BoNT/Hc or TeNT/Hc) derived from either their native genes or synthetic genes with codon usage optimised for expression in *E.coli* were amplified by PCR. Introduced *Bam*HI. (5') restriction sites and *Hind*III*, Sal*I or EcoRl (3') sites were used for most cloning operations with reading frames designed to start with the first base of the restriction site. Constructs were sequenced to confirm the presence of the correct sequence. The translocation domain of diphtheria toxin (DipT) was amplified from its native gene to introduce *Bam*HI and *Bgl*II sites at the 5' and 3' ends respectively. This *Bam*HI and *Bgl*II fragment was subcloned into the *Bam*HI site at the 5' end of the Hc fragment to generate an in-frame fusion. The entire heavy chain fragment (DipT-Hc) was excised as a *Bam*HI-*Hind*III or *Bam*HI*-Sal*I or *Bam*HI-*Eco*RI fragment and subcloned into a suitable expression vector.

Constructs for expression were subcloned into either an expression vector carrying a T7 polymerase promoter site (e.g. pET28, pET30 or derivatives (Novagen Inc, Madison, WI)) or to generate a fusion with maltose binding protein (e.g. pMALc2x (NEB)) as a suitable fragment. Clones with confirmed sequences were used to transform expression hosts: For T7 polymerase vectors *E.coli* BL21 (DE3) (Studier and Moffatt 1986 Journal of Molecular Biology 189:113-130) JM109 (DE3) or equivalent strains with a DE3 lysogen. For pMalc2x JM109, BL21, TG1, TB1 or other suitable expression strains.

The recombinant proteins expressed from pET vectors contain amino-terminal histidine (6-His) and T7 peptide tags allowing proteins to be purified by affinity chromatography on either a Cu²⁺ charged metal chelate column. Expression cultures were grown in Terrific Broth containing 30µg/ml kanamycin and 0.5% (w/v) glucose to an OD₆₀₀ of 2.0 and protein expression was induced with 500µM IPTG for 2 hours. Cells were lysed by either sonication or suitable detergent treatment (e.g. Bugbuster reagent; Novagen), cell debris pelleted by centrifugation and the supernatant loaded onto a metal chelate column charged with Cu²⁺ (Amersham-Pharmacia Biotech, Uppsala, Sweden). After loading proteins on the column and washing, proteins were eluted using imidazole. All buffers were used as specified by manufacturers. Where appropriate removal of the purification tag was carried out according to manufacturers instructions.

MBP fusions were purified on amylose resin columns as described by the manufacturer (NEB) following growth in Terrific Broth containing 100 µg/ml ampicillin and lysis as described above.

### 4. Purification of the heavy chain of botulinum Type C₁ neurotoxin.

Step 1. Botulinum type C₁ neurotoxin (5mg total) is dialysed against borate/phosphate buffer pH 8.5 (29 mM Na₂B₄O₇; 42 mM NaH₂PO₄ titrated to pH 8.5 with NaOH) and then applied (0.5ml min⁻¹) to a column (1cm x 5cm) of QAE-Sephadex (Pharmacia) equilibrated in the borate/phosphate pH 8.5 buffer. The column is then washed with a further 10ml of buffer followed by 10ml of the borate/phosphate buffer containing 10mM dithiothreitol. After the latter buffer has been allowed to run almost completely onto the column, 3ml of borate/phosphate pH 8.5 buffer containing 100mM dithiothreitol and 2M urea are carefully applied to the column and 2.5 ml of this buffer allowed to run onto the gel. The column is then sealed and incubated at 4°C overnight.

Step 2. The light subunit of the neurotoxin is eluted with 20ml of borate/phosphate pH 8.5 buffer containing 2M urea and 10mM dithiothreitol collecting 1.5ml fractions.

Step 3. The heavy subunit of the type C₁ neurotoxin is eluted with 20ml of borate/phosphate pH 8.5 buffer containing 2M urea, 10mM dithiothreitol and 0.2M NaCl. The presence of heavy chain in eluted fractions (1 ml) may be detected by uv measurement. Those fractions containing the highest concentration of protein are pooled and then dialysed against 0.05M Hepes pH 7.4 buffer containing 0.15M NaCl. The purified type C₁ neurotoxin may be stored frozen at -80°C until required.

### 5. Preparation of chemical C3-heavy chain conjugates.

Purified C3 was dialysed into phosphate buffered saline (PBS) pH7.4. Sulfo-LC-SPDP was added to give a final molar excess of 3:1 SPDP:C3 and reacted for 2 hours at room temperature. Free SPDP was removed by dialysis. Derivitised C3 was incubated with either native or recombinant heavy chain fragments containing a free cysteine residue for 20 hours at 4°C. Free C3 could be removed if required by cation exchange chromatography on a MonoS column run under the same conditions as the SP-Sepharose column described in example 1. Additional purification is also possible on a Cibacron-Blue affinity resin if required.

### 6 Assessment of activity of C3 conjugates on neuroblastoma cells.

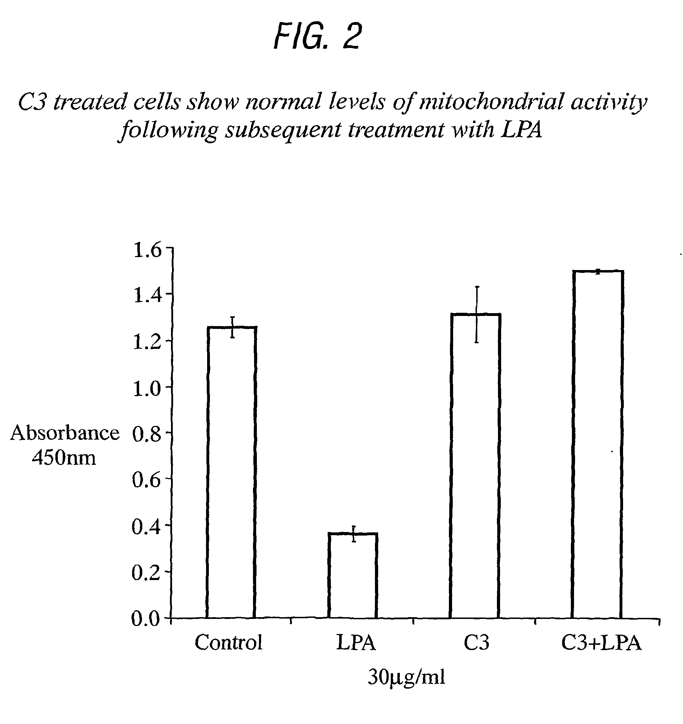

NG108 neuroblastoma cells were cultured in DMEM containing 10% foetal calf serum (FCS) and 2 mM glutamine at 37°C at 5% CO₂ saturation. Cells were plated onto poly-D-lysine coated 96 well culture plates at a density of 2x10⁴. After 24 hours the cells were changed into DMEM/glutamine medium lacking FCS and grown for a further 16 hours as above. Conjugate or free C3 was added and the cells incubated for either 3 hours or overnight. The cells were treated with 1µM lyso-phosphatidic acid (LPA) in DMEM/glutamine. Cells were observed for morphological changes under a microscope. As shown in Figure 1 C3 at a final concentration of 30µg/ml protected the cells from LPA mediated neurite retraction. To assay for cellular events in response to LPA treatment an MTT assay was used as described previously (Alley MC, Scudiero DA, Monks A, Hursey ML, Czerwinski MJ et al (1988) Feasibility of drug screening with panels of human tumor cell lines using a microculture tetrazolium assay. Cancer Res 48, 589-601). Assay results shown in Figure 2 show a marked reduction in mitochondrial activity, as measured by reduction of MTT, in LPA treated cells. Cells pre-treated with C3 (30µg/ml final concentration) before addition of LPA at showed similar levels of mitochondrial activity to untreated control cells indicating that C3 prevents LPA-induced cellular damage. This is indicative of protective or neurostimulatory effect in damaged neurons.

### Expressed Sequence identifications.

In the sequence listing accompanying this specification, the sequences have the following derivation:-
Sequence 1 C3 protein from *Clostridium botulinum* lacking the N-terminal signal sequence and including a factor Xa cleavge site immediately adjacent to the initial Alanine amino acid of the mature sequence.
Sequence 2 C3 protein from *Staphylococcus aureus* lacking the N-terminal signal sequence and including a factor Xa cleavage site immediately adjacent to the initial Alanine amino acid of the mature sequence. The protein refers to the C3Stau 2 isoform sometimes referred to as EDIN B
Sequence 3 Gene encoding C3 protein from *Staphylococcus aureus* lacking the N-terminal signal sequence and including a factor Xa cleavage site immediately adjacent to the initial Alanine amino acid of the mature sequence. The synthetic gene, with codon usage optimised for expression in *E.coli,* encodes the C3Stau 2 isoform sometimes referred to as EDIN B
Sequence 4 mature C3 protein from *Staphylococcus aureus;* termed C3Stau 1 (EDIN A)
Sequence 5 full length C3 protein from *Staphylococcus aureus;* termed C3Stau 1 (EDIN A) including a signal sequence at the N-terminus.
Sequence 6 mature C3 protein from *Clostridium limosum*
Sequence 7 C3-like protein from *Listeria monocytogenes*
Sequence 8 C3-like protein from *Clostridium acetobutylicum*
Sequence 9 C3-like protein from *Streptococcus pyogenes* (native protein sequence)
Sequence 10 Second C3-like protein from *Streptococcus pyogenes* (native protein sequence)
Sequence 11 shows the native heavy chain (HC) of BoNT/C1 with the dichain linker region. The native Factor Xa cleavage site within the linker region is also present. Optionally the fragment can also include the amino acids GS at the N-terminus, encoded by an in-frame *Bam*HI site used for cloning purposes.
Sequence 12 shows the isolated receptor binding domain H_{c} from BoNT/C1. This corresponds to the C-terminal domain of the H_{c} and has been shown to confer the ability to bind to neuronal receptors. Optionally the domain sequence is preceded at the N-terminus by the amino acids GS encoded for by an in-frame *Bam*HI site used for cloning of the fragment.
Sequence 13 In expression constructs the first alanine residue shown is preceded by a factor Xa cleavage site such that the proteolytic processing removes all of the fusion partner. The intrachain linker is simultaneously cleaved during the same processing step.
Sequence 14 shows the fusion protein C3Stau2 BoNT/C1- HC with the dichain linker region. In expression constructs the first alanine residue shown is preceded by a factor Xa cleavage site such that the proteolytic processing removes all of the fusion partner. The intrachain linker is simultaneously cleaved during the same processing step.
Sequence 15 shows the fusion protein C3bot BoNT/C1- H_{c} with the linker region derived from the native BoNT/C1. In expression constructs the first alanine residue shown is preceded by a factor Xa cleavage site such that the proteolytic processing removes all of the fusion partner. The intrachain linker is simultaneously cleaved during the same processing step. This construct differs from Sequence 13 in that it does not contain the H_{N} domain and as such lacks a translocation function. This alters the intracellular-trafficking of the C3 fragment in cells. Optionally the linker can be removed, with the C3 fragment joined directly onto the BoNT/Cl-H_{c} fragment. The GS amino acid residues following the linker sequence are derived from the *Bam*HI site used in the construction of the fragment.
Sequences 16- 24 show a variety of alternative linkers suitable for joining the C3 entity to the BoNT/C1 HC or H_{C} fragment. In variant embodiments, these linkers optionally replace the native linker sequence, identified as Sequence 22. In several instances these linkers can be cleaved by a different specific proteases allowing the sequential processing of the fusion site with Factor Xa and the intra-chain site.

## Claims

1. A conjugate, for delivery of a therapeutic agent to a neuronal cell, said conjugate comprising:
a therapeutic agent which inhibits at least one member of the Rho group of GTPases,
a translocation domain that is a domain or fragment of a protein that translocates the therapeutic agent into the cell, and
a neuronal cell targeting component, which component consists of a Hc domain of botulinum C1 toxin, or a fragment thereof which retains the binding affinity for neuronal cells of the native Hc domain,
and wherein the He domain or fragment thereof is made recombinantly.

2. A conjugate according to Claim 1 wherein the translocation domain is a clostridial translocation domain.

3. A conjugate according to Claim 1 wherein the translocation domain is a non-clostridial translocation domain.

4. A conjugate according to Claim 2 wherein the translocation domain is a C. botulinum, C. butylicum, C. argentinense or C. tetani translocation domain.

5. A conjugate according to Claim 3 wherein the translocation domain is a diphtheria toxin, Pseudomonas exotoxin A, influenza virus haemagglutinin fusogenic peptide or amphiphilic peptide translocation domain.

6. A conjugate according to Claim 1, wherein the translocation domain is selected from a botulinum C1 toxin and fragments thereof, and diphtheria toxin and fragments thereof.

7. A conjugate according to Claim 1 wherein the translocation domain is a membrane disrupting peptide.

8. A conjugate according Claim 1, wherein the therapeutic agent is selected from the group consisting of drugs, growth factors, enzymes, DNA, modified viruses, drug release systems, or a combination thereof.

9. A conjugate according to any preceding claim wherein the therapeutic agent is a C3 enzyme.

10. A conjugate according to Claim 9, wherein the C3 enzyme is a C. *botulinum, C. limosum, B. cereus, S. aureus, C. acetobutylicum, S. pyogenes, L. monocytogenes* C3 *enzyme.*

11. A conjugate according to Claim 9 wherein the C3 enzyme is selected from the group consisting of C3Stau2, C3Stau1, and C3bot.

12. A conjugate according to Claim 9 wherein the C3 enzyme is selected from SEQ ID Nos: 1-10.

13. A conjugate according to any preceding claim, wherein the therapeutic agent and the Hc domain are joined to each other directly or via a linker molecule.

14. A conjugate according to any of Claims 1-12 wherein the therapeutic agent, the Hc domain and the translocation domain are joined to each other directly or via a linker molecule.

15. A conjugate according to Claim 13 or 14, wherein the linker molecule is selected from the group consisting of (GGGGS)₂, (GGGGS)₃, the interdomain linker of cellulase, PPPIEGR, collagen-like spacer, trypsin-sensitive diphtheria toxin peptide, or SEQ ID Nos: 16-24.

16. A conjugate according to any preceding claim wherein the composition is a single polypeptide.

17. A conjugate according to any of Claims 1-15, wherein the composition is a dichain polypeptide.

18. A composition comprising a conjugate according to any preceding claim, wherein the composition is a suspension, emulsion, solution or a freeze-dried powder.

19. A composition according to Claim 18, wherein the conjugate is suspended or diluted in a pharmaceutically acceptable liquid.

20. A method of making a conjugate according to any of Claims 1-17 comprising expressing a DNA encoding the therapeutic agent, the translocation domain and the neuronal cell targeting domain.

21. Use of the conjugate according to any of Claims 1-17 or the composition according to Claim 18 or Claim 19, for the manufacture of a medicament for promoting nerve regeneration.

22. A conjugate according to any of Claims 1-17 or a composition according to Claim 18 or Claim 19, for use in promoting nerve regeneration.

## Patentansprüche

1. Konjugat zur Bereitstellung eines therapeutischen Mittels in einer neuronalen Zelle, wobei das Konjugat umfasst:
ein therapeutisches Mittel, welches mindestens einen Bestandteil der Rho-Gruppe von GTPasen inhibiert,
eine Translokationsdomäne, welche eine Domäne oder Fragment eines Proteins, welches das therapeutische Mittel in die Zelle translokiert, ist, und
eine Komponente zum Zielrichten auf neuronale Zellen, wobei die Komponente aus einer Hc-Domäne von Botulinum C1-Toxin oder einem Fragment davon, welches die Bindungsaffinität für neuronale Zellen der nativen Hc-Domäne beibehält, besteht,
und wobei die Hc-Domäne oder das Fragment davon rekombinant gemacht ist.

2. Konjugat gemäß Anspruch 1, wobei die Translokationsdomäne eine Clostridial-Translokationsdomäne ist.

3. Konjugat gemäß Anspruch 1, wobei die Translokationsdomäne eine Nicht-Clostridial-Translokationsdomäne ist.

4. Konjugat gemäß Anspruch 2, wobei die Translokationsdomäne eine C. botulinum-, C. butylicum-, C. argentinense- oder C. tetani-Translokationsdomäne ist.

5. Konjugat gemäß Anspruch 3, wobei die Translokationsdomäne eine Diphtherietoxin-, Pseudomonas-Exotoxin A-, zu Fusion fähiges Influenzavirus-Hämagglutininpeptid-oder amphiphiles Peptid-Translokationsdomäne ist.

6. Konjugat gemäß Anspruch 1, wobei die Translokationsdomäne aus einem Botulinum C1-Toxin und Fragmenten davon und Diphtherietoxin und Fragmenten davon ausgewählt ist.

7. Konjugat gemäß Anspruch 1, wobei die Translokationsdomäne ein Membranaufbrechendes Peptid ist.

8. Konjugat gemäß Anspruch 1, wobei das therapeutische Mittel aus der Gruppe, bestehend aus Arzneistoffen, Wachstumsfaktoren, Enzymen, DNA, modifizierten Viren, Arzneistofffreisetzungssystemen oder einer Kombination davon, ausgewählt ist.

9. Konjugate gemäß jedem vorhergehenden Anspruch, wobei das therapeutische Mittel ein C3-Enzym ist.

10. Konjugat gemäß Anspruch 9, wobei das C3-Enzym ein C. *botulinum-, C. limosum-, B. cereus, S. aureus, C. acetobutylicum-, S. pyogenes-, L. monocytogenes-C3-Enzym* ist.

11. Konjugat gemäß Anspruch 9, wobei das C3-Enzym aus der Gruppe, bestehend aus C3Stau2, C3Stau1 und C3bot, ausgewählt ist.

12. Konjugat gemäß Anspruch 9, wobei das C3-Enzym aus SEQ ID Nr.: 1 bis 10 ausgewählt ist.

13. Konjugat gemäß jedem vorhergehenden Anspruch, wobei das therapeutische Mittel und die Hc-Domäne direkt miteinander oder über ein Linkermolekül verbunden sind.

14. Konjugat gemäß jedem der Ansprüche 1 bis 12, wobei das therapeutische Mittel, die Hc-Domäne und die Translokationsdomäne direkt miteinander oder über ein Linkermolekül verbunden sind.

15. Konjugat gemäß Anspruch 13 oder 14, wobei das Linkermolekül aus der Gruppe, bestehend aus (GGGGS)₂, (GGGGS)₃, dem Zwischendomänenlinker von Cellulase, PPPIEGR, Kollagen-ähnlichen Spacer, Trypsin-empfindlichen Diphtherietoxinpeptid oder SEQ ID Nr.: 16 bis 24, ausgewählt ist.

16. Konjugat gemäß jedem vorhergehenden Anspruch, wobei die Zusammensetzung ein einzelnes Polypeptid ist.

17. Konjugat gemäß jedem der Ansprüche 1 bis 15, wobei die Zusammensetzung ein Zweiketten-Polypeptid ist.

18. Zusammensetzung, umfassend ein Konjugat gemäß jedem vorhergehenden Anspruch, wobei die Zusammensetzung eine Suspension, Emulsion, Lösung oder ein gefriergetrocknetes Pulver ist.

19. Zusammensetzung gemäß Anspruch 18, wobei das Konjugat in einer pharmazeutisch verträglichen Flüssigkeit suspendiert oder verdünnt ist.

20. Verfahren zur Herstellung eines Konjugats gemäß jedem der Ansprüche 1 bis 17, umfassend das Exprimieren einer DNA, welche das therapeutische Mittel, die Translokationsdomäne und die Domäne zum Zielrichten auf neuronale Zellen kodiert.

21. Verwendung des Konjugats gemäß jedem der Ansprüche 1 bis 17 oder der Zusammensetzung gemäß Anspruch 18 oder Anspruch 19 zur Herstellung eines Medikaments zur Förderung von Nervenregeneration.

22. Konjugat gemäß jedem der Ansprüche 1 bis 17 oder Zusammensetzung gemäß Anspruch 18 oder Anspruch 19 zur Verwendung bei der Förderung von Nervenregeneration.

## Revendications

1. Conjugué, pour la délivrance d'un agent thérapeutique à une cellule neuronale, ledit conjugué comprenant :
un agent thérapeutique qui inhibe au moins un membre du groupe Rho des GTPases,
un domaine de translocation qui est un domaine ou un fragment d'une protéine qui transloque l'agent thérapeutique dans la cellule, et
un composant ciblant les cellules neuronales, lequel composant est constitué d'un domaine Hc de la toxine botulinique C1, ou d'un fragment de celui-ci qui conserve l'affinité de liaison pour les cellules neuronales du domaine Hc natif,
et où le domaine Hc ou le fragment de celui-ci est fabriqué de manière recombinante.

2. Conjugué selon la revendication 1, dans lequel le domaine de translocation est un domaine de translocation clostridien.

3. Conjugué selon la revendication 1, dans lequel le domaine de translocation est un domaine de translocation non clostridien.

4. Conjugué selon la revendication 2, dans lequel le domaine de translocation est un domaine de translocation de C. botulinum, C. butylicum, C. argentinense ou C. tetani.

5. Conjugué selon la revendication 3, dans lequel le domaine de translocation est un domaine de translocation de la toxine diphtérique, de l'exotoxine A de Pseudomonas, du peptide fusogénique ou du peptide amphiphile de l'hémagglutinine du virus de la grippe.

6. Conjugué selon la revendication 1, dans lequel le domaine de translocation est choisi parmi une toxine botulinique C1 et des fragments de celle-ci et la toxine diphtérique et des fragments de celle-ci.

7. Conjugué selon la revendication 1, dans lequel le domaine de translocation est un peptide rompant la membrane.

8. Conjugué selon la revendication 1, dans lequel l'agent thérapeutique est choisi dans le groupe constitué de médicaments, de facteurs de croissance, d'enzymes, d'ADN, de virus modifiés, de systèmes de libération de médicaments, ou d'une combinaison de ceux-ci.

9. Conjugué selon l'une quelconque des revendications précédentes, dans lequel l'agent thérapeutique est une enzyme C3.

10. Conjugué selon la revendication 9, dans lequel l'enzyme C3 est une enzyme C3 de C. botulinum, C. limosum, B. cereus, S. aureus, C. acetobutylicum, S. pyogenes, L. monocytogenes.

11. Conjugué selon la revendication 9, dans lequel l'enzyme C3 est choisie dans le groupe constitué de C3Stau2, C3Staul et C3bot.

12. Conjugué selon la revendication 9, dans lequel l'enzyme C3 est choisie parmi les SEQ ID n° 1 à 10.

13. Conjugué selon l'une quelconque des revendications précédentes, dans lequel l'agent thérapeutique et le domaine Hc sont joints l'un à l'autre directement ou par l'intermédiaire d'une molécule de lieur.

14. Conjugué selon l'une quelconque des revendications 1 à 12, dans lequel l'agent thérapeutique, le domaine Hc et le domaine de translocation sont joints l'un à l'autre directement ou par l'intermédiaire d'une molécule de lieur.

15. Conjugué selon la revendication 13 ou 14, dans lequel la molécule de lieur est choisie dans le groupe constitué de (GGGGS)₂, (GGGGS)₃, le lieur interdomaine de cellulase, PPPIEGR, un espaceur de type collagène, un peptide de la toxine diphtérique sensible à la trypsine, ou les SEQ ID n° 16 à 24.

16. Conjugué selon l'une quelconque des revendications précédentes, où la composition est un polypeptide simple.

17. Conjugué selon l'une quelconque des revendications 1 à 15, où la composition est polypeptide bichaîne.

18. Composition comprenant un conjugué selon l'une quelconque des revendications précédentes, où la composition est une suspension, une émulsion, une solution ou une poudre lyophilisée.

19. Composition selon la revendication 18, dans laquelle le conjugué est en suspension ou dilué dans un liquide pharmaceutiquement acceptable.

20. Procédé de fabrication d'un conjugué selon l'une quelconque des revendications 1 à 17 comprenant l'expression d'un ADN codant pour l'agent thérapeutique, le domaine de translocation et le domaine de ciblage des cellules neuronales.

21. Utilisation du conjugué selon l'une quelconque des revendications 1 à 17, ou de la composition selon la revendication 18 ou la revendication 19, pour la fabrication d'un médicament destiné à favoriser la régénération des nerfs.

22. Conjugué selon l'une quelconque des revendications 1 à 17 ou composition selon la revendication 18 ou la revendication 19, pour une utilisation visant à favoriser la régénération des nerfs.


### SEQUENCE LISTING

<110> Health Protection Agency
   Sutton, John Shone, Clifford
<120> Targeted Agents for Nerve Regeneration
<130> GWS/CMS/24346WO
<150> GB 0216865.6
   <151> 2002-07-19
<160> 24
<170> PatentIn version 3.1
<210> 1
   <211> 215
   <212> PRT
   <213> Artificial Sequence
<220>
   <223> Synthetic
<400> 1
   Ile Glu Gly Arg Ala Tyr Ser Asn Thr Tyr Gln Glu Phe Thr Asn Ile
   1               5                   10                  15
   Asp Gln Ala Lys Ala Trp Gly Asn Ala Gln Tyr Lys Lys Tyr Gly Leu
               20                  25                  30
   Ser Lys Ser Glu Lys Glu Ala Ile Val Ser Tyr Thr Lys Ser Ala Ser
           35                  40                  45
   Glu Ile Asn Gly Lys Leu Arg Gln Asn Lys Gly Val Ile Asn Gly Phe
       50                  55                  60
   Pro Ser Asn Leu Ile Lys Gln Val Glu Leu Leu Asp Lys Ser Phe Asn
   65                  70                  75                  80
   Lys Met Lys Thr Pro Glu Asn Ile Met Leu Phe Arg Gly Asp Asp Pro
                   85                  90                  95
   Ala Tyr Leu Gly Thr Glu Phe Gln Asn Thr Leu Leu Asn Ser Asn Gly
               100                 105                 110
   Thr Ile Asn Lys Thr Ala Phe Glu Lys Ala Lys Ala Lys Phe Leu Asn
           115                 120                 125
   Lys Asp Arg Leu Glu Tyr Gly Tyr Ile Ser Thr Ser Leu Met Asn Val
       130                 135                 140
   Ser Gln Phe Ala Gly Arg Pro Ile Ile Thr Lys Phe Lys Val Ala Lys
   145 150 155 160
   Gly Ser Lys Ala Gly Tyr Ile Asp Pro Ile Ser Ala Phe Ala Gly Gln
   165 170 175
   Leu Glu Met Leu Leu Pro Arg His Ser Thr Tyr His Ile Asp Asp Met
   180 185 190
   Arg Leu Ser Ser Asp Gly Lys Gln Ile Ile Ile Thr Ala Thr Met Met
   195 200 205
   Gly Thr Ala Ile Asn Pro Lys
   210 215
<210> 2
   <211> 212
   <212> PRT
   <213> Artificial Sequence
<220>
   <223> Synthetic
<400> 2
   Ala Glu Thr Lys Asn Phe Thr Asp Leu Val Glu Ala Thr Lys Trp Gly
   1 5 10 15
   Asn Ser Leu Ile Lys Ser Ala Lys Tyr Ser Ser Lys Asp Lys Met Ala
   20 25 30
   Ile Tyr Asn Tyr Thr Lys Asn Ser Ser Pro Ile Asn Thr Pro Leu Arg
   35 40 45
   Ser Ala Asn Gly Asp Val Asn Lys Leu Ser Glu Asn Ile Gln Glu Gln
   50 55 60
   Val Arg Gln Leu Asp Ser Thr Ile Ser Lys Ser Val Thr Pro Asp Ser
   65 70 75 80
   Val Tyr Val Tyr Arg Leu Leu Asn Leu Asp Tyr Leu Ser Ser Ile Thr
   85 90 95
   Gly Phe Thr Arg Glu Asp Leu His Met Leu Gln Gln Thr Asn Asn Gly
   100 105 110
   Gln Tyr Asn Glu Ala Leu Val Ser Lys Leu Asn Asn Leu Met Asn Ser
   115 120 125
   Arg Ile Tyr Arg Glu Asn Gly Tyr Ser Ser Thr Gln Leu Val Ser Gly
   130 135 140
   Ala Ala Leu Ala Gly Arg Pro Ile Glu Leu Lys Leu Glu Leu Pro Lys
   145 150 155 160
   Gly Thr Lys Ala Ala Tyr Ile Asp Ser Lys Glu Leu Thr Ala Tyr Pro
   165 170 175
   Gly Gln Gln Glu Val Leu Leu Pro Arg Gly Thr Glu Tyr Ala Val Gly
   180 185 190
   Ser Val Lys Leu Ser Asp Asn Lys Arg Lys Ile Ile Ile Thr Ala Val
   195 200 205
   Val Phe Lys Lys
   210
<210> 3
   <211> 636
   <212> PRT
   <213> Artificial Sequence
<220>
   <223> Synthetic
<400> 3
   Gly Cys Thr Gly Ala Ala Ala Cys Cys Ala Ala Ala Ala Ala Cys Thr
   1               5                   10                  15
   Thr Cys Ala Cys Cys Gly Ala Cys Cys Thr Gly Gly Thr Thr Gly Ala
               20                  25                  30
   Ala Gly Cys Thr Ala Cys Cys Ala Ala Ala Thr Gly Gly Gly Gly Thr
           35                  40                  45
   Ala Ala Cys Thr Cys Thr Cys Thr Gly Ala Thr Cys Ala Ala Ala Thr
       50                  55                  60
   Cys Thr Gly Cys Thr Ala Ala Ala Thr Ala Cys Thr Cys Thr Thr Cys
   65                  70                  75                  80
   Thr Ala Ala Ala Gly Ala Cys Ala Ala Ala Ala Thr Gly Gly Cys Thr
                   85                  90                  95
   Ala Thr Cys Thr Ala Cys Ala Ala Cys Thr Ala Cys Ala Cys Cys Ala
               100                 105                 110
   Ala Ala Ala Ala Cys Thr Cys Thr Thr Cys Thr Cys Cys Gly Ala Thr
           115                 120                 125
   Cys Ala Ala Cys Ala Cys Cys Cys Cys Gly Cys Thr Gly Cys Gly Thr
       130                 135                 140
   Thr Cys Thr Gly Cys Thr Ala Ala Cys Gly Gly Thr Gly Ala Cys Gly
   145                 150                 155                 160
   Thr Thr Ala Ala Cys Ala Ala Ala Cys Thr Gly Thr Cys Thr Gly Ala
                   165                 170                 175
   Ala Ala Ala Cys Ala Thr Cys Cys Ala Gly Gly Ala Ala Cys Ala Gly
               180                 185                 190
   Gly Thr Thr Cys Gly Thr Cys Ala Gly Cys Thr Gly Gly Ala Cys Thr
           195                 200                 205
   Cys Thr Ala Cys Cys Ala Thr Cys Thr Cys Thr Ala Ala Ala Thr Cys
       210                 215                 220
   Thr Gly Thr Thr Ala Cys Cys Cys Cys Gly Gly Ala Cys Thr Cys Thr
   225                 230                 235                 240
   Gly Thr Thr Thr Ala Cys Gly Thr Thr Thr Ala Cys Cys Gly Thr Cys
                   245                 250                 255
   Thr Gly Cys Thr Gly Ala Ala Cys Cys Thr Gly Gly Ala Cys Thr Ala
               260                 265                 270
   Cys Cys Thr Gly Thr Cys Thr Thr Cys Thr Ala Thr Cys Ala Cys Cys
           275                 280                 285
   Gly Gly Thr Thr Thr Cys Ala Cys Cys Cys Gly Thr Gly Ala Ala Gly
       290                 295                 300
   Ala Cys Cys Thr Gly Cys Ala Cys Ala Thr Gly Cys Thr Gly Cys Ala
   305                 310                 315                 320
   Gly Cys Ala Gly Ala Cys Cys Ala Ala Cys Ala Ala Cys Gly Gly Thr
                   325                 330                 335
   Cys Ala Gly Thr Ala Cys Ala Ala Cys Gly Ala Ala Gly Cys Thr Cys
               340                 345                 350
   Thr Gly Gly Thr Thr Thr Cys Thr Ala Ala Ala Cys Thr Gly Ala Ala
           355                 360                 365
   Cys Ala Ala Cys Cys Thr Gly Ala Thr Gly Ala Ala Cys Thr Cys Thr
       370                 375                 380
   Cys Gly Thr Ala Thr Cys Thr Ala Cys Cys Gly Thr Gly Ala Ala Ala
   385                 390                 395                 400
   Ala Cys Gly Gly Thr Thr Ala Cys Thr Cys Thr Thr Cys Thr Ala Cys
                   405                 410                 415
   Cys Cys Ala Gly Cys Thr Gly Gly Thr Thr Thr Cys Thr Gly Gly Thr
               420                 425                 430
   Gly Cys Thr Gly Cys Thr Cys Thr Gly Gly Cys Thr Gly Gly Thr Cys
   435 440 445
   Gly Thr Cys Cys Gly Ala Thr Cys Gly Ala Ala Cys Thr Gly Ala Ala
   450 455 460
   Ala Cys Thr Gly Gly Ala Ala Cys Thr Gly Cys Cys Gly Ala Ala Ala
   465 470 475 480
   Gly Gly Thr Ala Cys Cys Ala Ala Ala Gly Cys Thr Gly Cys Thr Thr
   485 490 495
   Ala Cys Ala Thr Cys Gly Ala Cys Thr Cys Thr Ala Ala Ala Gly Ala
   500 505 510
   Ala Cys Thr Gly Ala Cys Cys Gly Cys Thr Thr Ala Cys Cys Cys Cys
   515 520 525
   Gly Gly Thr Cys Ala Gly Cys Ala Gly Gly Ala Ala Gly Thr Thr Cys
   530 535 540
   Thr Gly Cys Thr Gly Cys Cys Gly Cys Gly Thr Gly Gly Thr Ala Cys
   545 550 555 560
   Cys Gly Ala Ala Thr Ala Cys Gly Cys Thr Gly Thr Thr Gly Gly Thr
   565 570 575
   Thr Cys Thr Gly Thr Thr Ala Ala Ala Cys Thr Gly Thr Cys Thr Gly
   580 585 590
   Ala Cys Ala Ala Cys Ala Ala Ala Cys Gly Thr Ala Ala Ala Ala Thr
   595 600 605
   Cys Ala Thr Cys Ala Thr Cys Ala Cys Cys Gly Cys Thr Gly Thr Thr
   610 615 620
   Gly Thr Thr Thr Thr Cys Ala Ala Gly Ala Ala Gly
   625 630 635
<210> 4
   <211> 212
   <212> PRT
   <213> Staphylococcus aureus
<400> 4
   Ala Asp Val Lys Asn Phe Thr Asp Leu Asp Glu Ala Thr Lys Trp Gly
   1               5                   10                  15
   Asn Lys Leu Ile Lys Gln Ala Lys Tyr Ser Ser Asp Asp Lys Ile Ala
               20                  25                  30
   Leu Tyr Glu Tyr Thr Lys Asp Ser Ser Lys Ile Asn Gly Pro Leu Arg
           35                  40                  45
   Leu Ala Gly Gly Asp Ile Asn Lys Leu Asp Ser Thr Thr Gln Asp Lys
       50                  55                  60
   Val Arg Arg Leu Asp Ser Ser Ile Ser Lys Ser Thr Thr Pro Glu Ser
   65                  70                  75                  80
   Val Tyr Val Tyr Arg Leu Leu Asn Leu Asp Tyr Leu Thr Ser Ile Val
                   85                  90                  95
   Gly Phe Thr Asn Glu Asp Leu Tyr Lys Leu Gln Gln Thr Asn Asn Gly
               100                 105                 110
   Gln Tyr Asp Glu Asn Leu Val Arg Lys Leu Asn Asn Val Met Asn Ser
           115                 120                 125
   Arg Ile Tyr Arg Glu Asp Gly Tyr Ser Ser Thr Gln Leu Val Ser Gly
       130                 135                 140
   Ala Ala Val Gly Gly Arg Pro Ile Glu Leu Arg Leu Glu Leu Pro Lys
   145                 150                 155                 160
   Gly Thr Lys Ala Ala Tyr Leu Asn Ser Lys Asp Leu Thr Ala Tyr Tyr
                   165                 170                 175
   Gly Gln Gln Glu Val Leu Leu Pro Arg Gly Thr Glu Tyr Ala Val Gly
               180                 185                 190
   Ser Val Glu Leu Ser Asn Asp Lys Lys Lys Ile Ile Ile Thr Ala Ile
           195                 200                 205
   Val Phe Lys Lys
       210
<210> 5
   <211> 247
   <212> PRT
   <213> Staphylococcus aureus
<400> 5
   Met Lys Arg Lys Leu Phe Phe Lys Ile Ile Phe Val Leu Ser Leu Val
   1               5                   10                  15
   Leu Ser Ile His Ser Ile Asn Asp Arg Thr Thr Glu Leu Ser Asn Ile
               20                  25                  30
   Ala Leu Ala Asp Asp Val Lys Asn Phe Thr Asp Leu Thr Glu Ala Thr
           35                  40                  45
   Asn Trp Gly Asn Lys Leu Ile Lys Gln Ala Asn Tyr Ser Ser Lys Asp
       50                  55                  60
   Lys Glu Ala Ile Tyr Asn Tyr Thr Lys Tyr Ser Ser Pro Ile Asn Thr
   65                  70                  75                  80
   Pro Leu Arg Ser Ser Gln Gly Asp Ile Ser Asn Phe Ser Ala Asp Leu
                   85                  90                  95
   Gln Glu Lys Ile Leu Arg Leu Asp Arg Leu Ile Ser Lys Ser Ser Thr
               100                 105                 110
   Ser Asp Ser Val Tyr Val Tyr Arg Leu Leu Asn Leu Asp Tyr Leu Ser
           115                 120                 125
   Ser Val Lys Gly Phe Ser Ser Glu Asp Leu Glu Leu Leu Tyr Lys Thr
       130                 135                 140
   Glu Asn Gly Lys Tyr Asn Glu Glu Leu Val Lys Lys Leu Asn Asn Ile
   145                 150                 155                 160
   Met Asn Ser Lys Ile Tyr Thr Glu Tyr Gly Tyr Ser Ser Thr Gln Leu
                   165                 170                 175
   Val Lys Gly Ala Ala Leu Ala Gly Arg Pro Ile Glu Leu Lys Leu Gln
               180                 185                 190
   Leu Pro Lys Gly Thr Lys Ala Ala Tyr Ile Asp Ser Lys Asn Leu Thr
           195                 200                 205
   Ala Tyr Pro Gly Gln Gln Glu Ile Leu Leu Pro Arg Gly Thr Asp Tyr
       210                 215                 220
   Thr Ile Asn Thr Val Lys Leu Ser Asp Asp His Lys Arg Ile Leu Ile
   225                 230                 235                 240
   Glu Gly Ile Val Phe Lys Lys
                   245
<210> 6
   <211> 211
   <212> PRT
   <213> Clostridium limosum
<400> 6
   Ala Tyr Ser Asn Thr Tyr Gln Glu Phe Thr Asn Ile Asp Gln Ala Lys
   1               5                   10                  15
   Ala Trp Gly Asn Ala Gln Tyr Lys Lys Tyr Gly Leu Ser Lys Ser Glu
               20                  25                  30
   Lys Glu Ala Ile Val Ser Tyr Thr Lys Ser Ala Ser Glu Ile Asn Gly
           35                  40                  45
   Lys Leu Arg Gln Asn Lys Gly Val Ile Asn Gly Phe Pro Ser Asn Leu
       50                  55                  60
   Ile Lys Gln Val Glu Leu Leu Asp Lys Ser Phe Asn Lys Met Lys Thr
   65                  70                  75                  80
   Pro Glu Asn Ile Met Leu Phe Arg Gly Asp Asp Pro Ala Tyr Leu Gly
                   85                  90                  95
   Thr Glu Phe Gln Asn Thr Leu Leu Asn Ser Asn Gly Thr Ile Asn Lys
               100                 105                 110
   Thr Ala Phe Glu Lys Ala Lys Ala Lys Phe Leu Asn Lys Asp Arg Leu
           115                 120                 125
   Glu Tyr Gly Tyr Ile Ser Thr Ser Leu Met Asn Val Ser Gln Phe Ala
       130                 135                 140
   Gly Arg Pro Ile Ile Thr Lys Phe Lys Val Ala Lys Gly Ser Lys Ala
   145                 150                 155                 160
   Gly Tyr Ile Asp Pro Ile Ser Ala Phe Ala Gly Gln Leu Glu Met Leu
                   165                 170                 175
   Leu Pro Arg His Ser Thr Tyr His Ile Asp Asp Met Arg Leu Ser Ser
               180                 185                 190
   Asp Gly Lys Gln Ile Ile Ile Thr Ala Thr Met Met Gly Thr Ala Ile
           195                 200                 205
   Asn Pro Lys
       210
<210> 7
   <211> 160
   <212> PRT
   <213> Listeria monocytogenes
<400> 7
   Asn Lys Ser Leu Lys Phe Thr Ser Leu Glu Glu Ser Glu Lys Trp Gly
   1               5                   10                  15
   Ile Asp Gly Phe Ser Val Trp Arg Asn Ser Leu Ser Ser Arg Glu Ile
               20                  25                  30
   Gln Ala Ile Arg Asp Tyr Thr Asp Ile Trp His Tyr Gly Asn Met Asn
           35                  40                  45
   Gly Tyr Leu Arg Gly Ser Val Glu Lys Leu Ala Pro Asp Asn Ala Glu
       50                  55                  60
   Arg Ile Lys Asn Leu Ser Ser Ala Leu Glu Lys Ala Glu Leu Pro Asp
   65                  70                  75                  80
   Asn Ile Ile Leu Tyr Arg Gly Thr Ser Ser Glu Ile Leu Asp Asn Phe
                   85                  90                  95
   Leu Asp Leu Lys Asn Leu Asn Tyr Gln Asn Leu Val Gly Lys Thr Ile
               100                 105                 110
   Glu Glu Lys Gly Phe Met Ser Thr Thr Thr Ile Ser Asn Gln Thr Phe
           115                 120                 125
   Ser Gly Asn Val Thr Met Lys Ile Asn Ala Pro Lys Gly Ser Lys Gly
       130                 135                 140
   Ala Tyr Leu Ala His Phe Ser Glu Thr Pro Glu Glu Ala Glu Val Leu
   145                 150                 155                 160
<210> 8
   <211> 175
   <212> PRT
   <213> Clostridium:acetobutylicum
<400> 8
   Thr Asn Met Asp Gln Ala Asn Glu Trp Gly Ser Gln Tyr Tyr Asp Asn
   1               5                   10                  15
   Trp Leu Lys Ser Leu Asn Asp Ser Glu Arg Asn Ala Ile Arg Gln Tyr
               20                  25                  30
   Thr Gly Asn Asp Tyr Lys Lys Ile Asn Asn Tyr Leu Arg Gly Val Asn
           35                  40                  45
   Asp Ser Leu Asp Gly Ile Asp Pro Lys Ile Ile Glu Asp Ile Lys Ser
       50                  55                  60
   Gly Leu Lys Lys Ala Ser Val Pro His Asp Met Lys Val Tyr Arg Gly
   65                  70                  75                  80
   Thr Asp Leu Asn Pro Leu Arg Asn Leu Ile Asp Val Gly Lys Asp Gly
                   85                  90                  95
   Ser Leu Asp Phe Ser Leu Val Gly Lys Thr Phe Lys Asp Asp Gly Phe
               100                 105                 110
   Met Ser Thr Ala Leu Val Lys Glu Ser Ser Phe Asp Tyr Met Asn Val
           115                 120                 125
   Ser Trp Glu Ile Asn Val Pro Lys Gly Thr Glu Ala Ala Tyr Val Ser
       130                 135                 140
   Lys Ile Ser Tyr Phe Pro Asp Glu Ala Glu Leu Leu Leu Asn His Gly
   145                 150                 155                 160
   Gln Glu Met Ile Ile Lys Glu Ala Thr Val Gly Ser Asp Gly Lys
                   165                 170                 175
<210> 9
   <211> 250
   <212> PRT
   <213> Streptococcus pyogenes
<400> 9
   Met Leu Lys Lys Arg Tyr Gln Leu Ala Ile Val Leu Leu Leu Ser Cys
   1               5                   10                  15
   Phe Ser Leu Ile Trp Gln Thr Glu Gly Leu Val Glu Leu Phe Val Cys
               20                  25                  30
   Glu His Tyr Glu Arg Ala Val Cys Glu Gly Thr Pro Ala Tyr Phe Thr
           35                  40                  45
   Phe Ser Asp Gln Lys Gly Ala Glu Thr Leu Ile Lys Lys Arg Trp Gly
       50                  55                  60
   Lys Gly Leu Ile Tyr Pro Arg Ala Glu Gln Glu Ala Met Ala Ala Tyr
   65                  70                  75                  80
   Thr Cys Gln Gln Ala Gly Pro Ile Asn Thr Ser Leu Asp Lys Ala Lys
                   85                  90                  95
   Gly Glu Leu Ser Gln Leu Thr Pro Glu Leu Arg Asp Gln Val Ala Gln
               100                 105                 110
   Leu Asp Ala Ala Thr His Arg Leu Val Ile Pro Trp Asn Ile Val Val
           115                 120                 125
   Tyr Arg Tyr Val Tyr Glu Thr Phe Leu Arg Asp Ile Gly Val Ser His
       130                 135                 140
   Ala Asp Leu Thr Ser Tyr Tyr Arg Asn His Gln Phe Asp Pro His Ile
   145                 150                 155                 160
   Leu Cys Lys Ile Lys Leu Gly Thr Arg Tyr Thr Lys His Ser Phe Met
                   165                 170                 175
   Ser Thr Thr Ala Leu Lys Asn Gly Ala Met Thr His Arg Pro Val Glu
               180                 185                 190
   Val Arg Ile Cys Val Lys Lys Gly Ala Lys Ala Ala Phe Val Glu Pro
           195                 200                 205
   Tyr Ser Ala Val Pro Ser Glu Val Glu Leu Leu Phe Pro Arg Gly Cys
       210                 215                 220
   Gln Leu Glu Val Val Gly Ala Tyr Val Ser Gln Asp Gln Lys Lys Leu
   225                 230                 235                 240
   His Ile Glu Ala Tyr Phe Lys Gly Ser Leu
                   245                 250
<210> 10
   <211> 250
   <212> PRT
   <213> Streptococcus pyogenes
<400> 10
   Met Leu Lys Lys Arg Tyr Gln Leu Ala Ile Val Leu Leu Leu Ser Cys
   1               5                   10                  15
   Phe Ser Leu Ile Trp Gln Thr Glu Gly Leu Val Glu Leu Phe Val Cys
               20                  25                  30
   Glu His Tyr Glu Arg Ala Val Cys Glu Gly Thr Pro Ala Tyr Phe Thr
           35                  40                  45
   Phe Ser Asp Gln Lys Gly Ala Glu Thr Leu Ile Lys Lys Arg Trp Gly
       50                  55                  60
   Lys Gly Leu Ile Tyr Pro Arg Ala Glu Gln Glu Ala Met Ala Ala Tyr
   65                  70                  75                  80
   Thr Cys Gln Gln Ala Gly Pro Ile Asn Thr Ser Leu Asp Lys Ala Lys
                   85                  90                  95
   Gly Glu Leu Ser Gln Leu Thr Pro Glu Leu Arg Asp Gln Val Ala Gln
               100                 105                 110
   Leu Asp Ala Ala Thr His Arg Leu Val Ile Pro Trp Asn Ile Val Val
           115                 120                 125
   Tyr Arg Tyr Val Tyr Glu Thr Phe Leu Arg Asp Ile Gly Val Ser His
       130                 135                 140
   Ala Asp Leu Thr Ser Tyr Tyr Arg Asn His Gln Phe Asp Pro His Ile
   145                 150                 155                 160
   Leu Cys Lys Ile Lys Leu Gly Thr Arg Tyr Thr Lys His Ser Phe Met
                   165                 170                 175
   Ser Thr Thr Ala Leu Lys Asn Gly Ala Met Thr His Arg Pro Val Glu
               180                 185                 190
   Val Arg Ile Cys Val Lys Lys Gly Ala Lys Ala Ala Phe Val Glu Pro
           195                 200                 205
   Tyr Ser Ala Val Pro Ser Glu Val Glu Leu Leu Phe Pro Arg Gly Cys
       210                 215                 220
   Gln Leu Glu Val Val Gly Ala Tyr Val Ser Gln Asp Gln Lys Lys Leu
   225                 230                 235                 240
   His Ile Glu Ala Tyr Phe Lys Gly Ser Leu
                   245                 250
<210> 11
   <211> 855
   <212> PRT
   <213> Clostridium botulinum
<400> 11
   Cys His Lys Ala Ile Asp Gly Arg Ser Leu Tyr Asn Lys Thr Leu Asp
   1               5                   10                  15
   Cys Arg Glu Leu Leu Val Lys Asn Thr Asp Leu Pro Phe Ile Gly Asp
               20                  25                  30
   Ile Ser Asp Val Lys Thr Asp Ile Phe Leu Arg Lys Asp Ile Asn Glu
           35                  40                  45
   Glu Thr Glu Val Ile Tyr Tyr Pro Asp Asn Val Ser Val Asp Gln Val
       50                  55                  60
   Ile Leu Ser Lys Asn Thr Ser Glu His Gly Gln Leu Asp Leu Leu Tyr
   65                  70                  75                  80
   Pro Ser Ile Asp Ser Glu Ser Glu Ile Leu Pro Gly Glu Asn Gln Val
                   85                  90                  95
   Phe Tyr Asp Asn Arg Thr Gln Asn Val Asp Tyr Leu Asn Ser Tyr Tyr
               100                 105                 110
   Tyr Leu Glu Ser Gln Lys Leu Ser Asp Asn Val Glu Asp Phe Thr Phe
           115                 120                 125
   Thr Arg Ser Ile Glu Glu Ala Leu Asp Asn Ser Ala Lys Val Tyr Thr
       130                 135                 140
   Tyr Phe Pro Thr Leu Ala Asn Lys Val Asn Ala Gly Val Gln Gly Gly
   145                 150                 155                 160
   Leu Phe Leu Met Trp Ala Asn Asp Val Val Glu Asp Phe Thr Thr Asn
   165 170 175
   Ile Leu Arg Lys Asp Thr Leu Asp Lys Ile Ser Asp Val Ser Ala Ile
   180 185 190
   Ile Pro Tyr Ile Gly Pro Ala Leu Asn Ile Ser Asn Ser Val Arg Arg
   195 200 205
   Gly Asn Phe Thr Glu Ala Phe Ala Val Thr Gly Val Thr Ile Leu Leu
   210 215 220
   Glu Ala Phe Pro Glu Phe Thr Ile Pro Ala Leu Gly Ala Phe Val Ile
   225 230 235 240
   Tyr Ser Lys Val Gln Glu Arg Asn Glu Ile Ile Lys Thr Ile Asp Asn
   245 250 255
   Cys Leu Glu Gln Arg Ile Lys Arg Trp Lys Asp Ser Tyr Glu Trp Met
   260 265 270
   Met Gly Thr Trp Leu Ser Arg Ile Ile Thr Gln Phe Asn Asn Ile Ser
   275 280 285
   Tyr Gln Met Tyr Asp Ser Leu Asn Tyr Gln Ala Gly Ala Ile Lys Ala
   290 295 300
   Lys Ile Asp Leu Glu Tyr Lys Lys Tyr Ser Gly Ser Asp Lys Glu Asn
   305 310 315 320
   Ile Lys Ser Gln Val Glu Asn Leu Lys Asn Ser Leu Asp Val Lys Ile
   325 330 335
   Ser Glu Ala Met Asn Asn Ile Asn Lys Phe Ile Arg Glu Cys Ser Val
   340 345 350
   Thr Tyr Leu Phe Lys Asn Met Leu Pro Lys Val Ile Asp Glu Leu Asn
   355 360 365
   Glu Phe Asp Arg Asn Thr Lys Ala Lys Leu Ile Asn Leu Ile Asp Ser
   370 375 380
   His Asn Ile Ile Leu Val Gly Glu Val Asp Lys Leu Lys Ala Lys Val
   385 390 395 400
   Asn Asn Ser Phe Gln Asn Thr Ile Pro Phe Asn Ile Phe Ser Tyr Thr
   405 410 415
   Asn Asn Ser Leu Leu Lys Asp Ile Ile Asn Glu Tyr Phe Asn Asn Ile
   420 425 430
   Asn Asp Ser Lys Ile Leu Ser Leu Gln Asn Arg Lys Asn Thr Leu Val
   435 440 445
   Asp Thr Ser Gly Tyr Asn Ala Glu Val Ser Glu Glu Gly Asp Val Gln
       450                 455                 460
   Leu Asn Pro Ile Phe Pro Phe Asp Phe Lys Leu Gly Ser Ser Gly Glu
   465                 470                 475                 480
   Asp Arg Gly Lys Val Ile Val Thr Gln Asn Glu Asn Ile Val Tyr Asn
                   485                 490                 495
   Ser Met Tyr Glu Ser Phe Ser Ile Ser Phe Trp Ile Arg Ile Asn Lys
               500                 505                 510
   Trp Val Ser Asn Leu Pro Gly Tyr Thr Ile Ile Asp Ser Val Lys Asn
           515                 520                 525
   Asn Ser Gly Trp Ser Ile Gly Ile Ile Ser Asn Phe Leu Val Phe Thr
       530                 535                 540
   Leu Lys Gln Asn Glu Asp Ser Glu Gln Ser Ile Asn Phe Ser Tyr Asp
   545                 550                 555                 560
   Ile Ser Asn Asn Ala Pro Gly Tyr Asn Lys Trp Phe Phe Val Thr Val
                   565                 570                 575
   Thr Asn Asn Met Met Gly Asn Met Lys Ile Tyr Ile Asn Gly Lys Leu
               580                 585                 590
   Ile Asp Thr Ile Lys Val Lys Glu Leu Thr Gly Ile Asn Phe Ser Lys
           595                 600                 605
   Thr Ile Thr Phe Glu Ile Asn Lys Ile Pro Asp Thr Gly Leu Ile Thr
       610                 615                 620
   Ser Asp Ser Asp Asn Ile Asn Met Trp Ile Arg Asp Phe Tyr Ile Phe
   625                 630                 635                 640
   Ala Lys Glu Leu Asp Gly Lys Asp Ile Asn Ile Leu Phe Asn Ser Leu
                   645                 650                 655
   Gln Tyr Thr Asn Val Val Lys Asp Tyr Trp Gly Asn Asp Leu Arg Tyr
               660                 665                 670
   Asn Lys Glu Tyr Tyr Met Val Asn Ile Asp Tyr Leu Asn Arg Tyr Met
           675                 680                 685
   Tyr Ala Asn Ser Arg Gln Ile Val Phe Asn Thr Arg Arg Asn Asn Asn
       690                 695                 700
   Asp Phe Asn Glu Gly Tyr Lys Ile Ile Ile Lys Arg Ile Arg Gly Asn
   705                 710                 715                 720
   Thr Asn Asp Thr Arg Val Arg Gly Gly Asp Ile Leu Tyr Phe Asp Met
                   725                 730                 735
   Thr Ile Asn Asn Lys Ala Tyr Asn Leu Phe Met Lys Asn Glu Thr Met
               740                 745                 750
   Tyr Ala Asp Asn His Ser Thr Glu Asp Ile Tyr Ala Ile Gly Leu Arg
           755                 760                 765
   Glu Gln Thr Lys Asp Ile Asn Asp Asn Ile Ile Phe Gln Ile Gln Pro
       770                 775                 780
   Met Asn Asn Thr Tyr Tyr Tyr Ala Ser Gln Ile Phe Lys Ser Asn Phe
   785                 790                 795                 800
   Asn Gly Glu Asn Ile Ser Gly Ile Cys Ser Ile Gly Thr Tyr Arg Phe
                   805                 810                 815
   Arg Leu Gly Gly Asp Trp Tyr Arg His Asn Tyr Leu Val Pro Thr Val
               820                 825                 830
   Lys Gln Gly Asn Tyr Ala Ser Leu Leu Glu Ser Thr Ser Thr His Trp
           835                 840                 845
   Gly Phe Val Pro Val Ser Glu
       850                 855
<210> 12
   <211> 454
   <212> PRT
   <213> Clostridium botulinum
<400> 12
   Gly Ser Phe Gln Asn Thr Ile Pro Phe Asn Ile Phe Ser Tyr Thr Asn
   1               5                   10                  15
   Asn Ser Leu Leu Lys Asp Ile Ile Asn Glu Tyr Phe Asn Asn Ile Asn
               20                  25                  30
   Asp Ser Lys Ile Leu Ser Leu Gln Asn Arg Lys Asn Thr Leu Val Asp
           35                  40                  45
   Thr Ser Gly Tyr Asn Ala Glu Val Ser Glu Glu Gly Asp Val Gln Leu
       50                  55                  60
   Asn Pro Ile Phe Pro Phe Asp Phe Lys Leu Gly Ser Ser Gly Glu Asp
   65                  70                  75                  80
   Arg Gly Lys Val Ile Val Thr Gln Asn Glu Asn Ile Val Tyr Asn Ser
                   85                  90                  95
   Met Tyr Glu Ser Phe Ser Ile Ser Phe Trp Ile Arg Ile Asn Lys Trp
               100                 105                 110
   Val Ser Asn Leu Pro Gly Tyr Thr Ile Ile Asp Ser Val Lys Asn Asn
           115                 120                 125
   Ser Gly Trp Ser Ile Gly Ile Ile Ser Asn Phe Leu Val Phe Thr Leu
       130                 135                 140
   Lys Gln Asn Glu Asp Ser Glu Gln Ser Ile Asn Phe Ser Tyr Asp Ile
   145                 150                 155                 160
   Ser Asn Asn Ala Pro Gly Tyr Asn Lys Trp Phe Phe Val Thr Val Thr
                   165                 170                 175
   Asn Asn Met Met Gly Asn Met Lys Ile Tyr Ile Asn Gly Lys Leu Ile
               180                 185                 190
   Asp Thr Ile Lys Val Lys Glu Leu Thr Gly Ile Asn Phe Ser Lys Thr
           195                 200                 205
   Ile Thr Phe Glu Ile Asn Lys Ile Pro Asp Thr Gly Leu Ile Thr Ser
       210                 215                 220
   Asp Ser Asp Asn Ile Asn Met Trp Ile Arg Asp Phe Tyr Ile Phe Ala
   225                 230                 235                 240
   Lys Glu Leu Asp Gly Lys Asp Ile Asn Ile Leu Phe Asn Ser Leu Gln
                   245                 250                 255
   Tyr Thr Asn Val Val Lys Asp Tyr Trp Gly Asn Asp Leu Arg Tyr Asn
               260                 265                 270
   Lys Glu Tyr Tyr Met Val Asn Ile Asp Tyr Leu Asn Arg Tyr Met Tyr
           275                 280                 285
   Ala Asn Ser Arg Gln Ile Val Phe Asn Thr Arg Arg Asn Asn Asn Asp
       290                 295                 300
   Phe Asn Glu Gly Tyr Lys Ile Ile Ile Lys Arg Ile Arg Gly Asn Thr
   305                 310                 315                 320
   Asn Asp Thr Arg Val Arg Gly Gly Asp Ile Leu Tyr Phe Asp Met Thr
                   325                 330                 335
   Ile Asn Asn Lys Ala Tyr Asn Leu Phe Met Lys Asn Glu Thr Met Tyr
               340                 345                 350
   Ala Asp Asn His Ser Thr Glu Asp Ile Tyr Ala Ile Gly Leu Arg Glu
           355                 360                 365
   Gln Thr Lys Asp Ile Asn Asp Asn Ile Ile Phe Gln Ile Gln Pro Met
       370                 375                 380
   Asn Asn Thr Tyr Tyr Tyr Ala Ser Gln Ile Phe Lys Ser Asn Phe Asn
   385 390 395 400
   Gly Glu Asn Ile Ser Gly Ile Cys Ser Ile Gly Thr Tyr Arg Phe Arg
   405 410 415
   Leu Gly Gly Asp Trp Tyr Arg His Asn Tyr Leu Val Pro Thr Val Lys
   420 425 430
   Gln Gly Asn Tyr Ala Ser Leu Leu Glu Ser Thr Ser Thr His Trp Gly
   435 440 445
   Phe Val Pro Val Ser Glu
   450
<210> 13
   <211> 1066
   <212> PRT
   <213> Artificial Sequence
<220>
   <223> Synthetic
<400> 13
   Ala Tyr Ser Asn Thr Tyr Gln Glu Phe Thr Asn Ile Asp Gln Ala Lys
   1               5                   10                  15
   Ala Trp Gly Asn Ala Gln Tyr Lys Lys Tyr Gly Leu Ser Lys Ser Glu
               20                  25                  30
   Lys Glu Ala Ile Val Ser Tyr Thr Lys Ser Ala Ser Glu Ile Asn Gly
           35                  40                  45
   Lys Leu Arg Gln Asn Lys Gly Val Ile Asn Gly Phe Pro Ser Asn Leu
       50                  55                  60
   Ile Lys Gln Val Glu Leu Leu Asp Lys Ser Phe Asn Lys Met Lys Thr
   65                  70                  75                  80
   Pro Glu Asn Ile Met Leu Phe Arg Gly Asp Asp Pro Ala Tyr Leu Gly
                   85                  90                  95
   Thr Glu Phe Gln Asn Thr Leu Leu Asn Ser Asn Gly Thr Ile Asn Lys
               100                 105                 110
   Thr Ala Phe Glu Lys Ala Lys Ala Lys Phe Leu Asn Lys Asp Arg Leu
           115                 120                 125
   Glu Tyr Gly Tyr Ile Ser Thr Ser Leu Met Asn Val Ser Gln Phe Ala
       130                 135                 140
   Gly Arg Pro Ile Ile Thr Lys Phe Lys Val Ala Lys Gly Ser Lys Ala
   145                 150                 155                 160
   Gly Tyr Ile Asp Pro Ile Ser Ala Phe Ala Gly Gln Leu Glu Met Leu
                   165                 170                 175
   Leu Pro Arg His Ser Thr Tyr His Ile Asp Asp Met Arg Leu Ser Ser
               180                 185                 190
   Asp Gly Lys Gln Ile Ile Ile Thr Ala Thr Met Met Gly Thr Ala Ile
           195                 200                 205
   Asn Pro Lys Cys His Lys Ala Ile Asp Gly Arg Ser Leu Tyr Asn Lys
       210                 215                 220
   Thr Leu Asp Cys Arg Glu Leu Leu Val Lys Asn Thr Asp Leu Pro Phe
   225                 230                 235                 240
   Ile Gly Asp Ile Ser Asp Val Lys Thr Asp Ile Phe Leu Arg Lys Asp
                   245                 250                 255
   Ile Asn Glu Glu Thr Glu Val Ile Tyr Tyr Pro Asp Asn Val Ser Val
               260                 265                 270
   Asp Gln Val Ile Leu Ser Lys Asn Thr Ser Glu His Gly Gln Leu Asp
           275                 280                 285
   Leu Leu Tyr Pro Ser Ile Asp Ser Glu Ser Glu Ile Leu Pro Gly Glu
       290                 295                 300
   Asn Gln Val Phe Tyr Asp Asn Arg Thr Gln Asn Val Asp Tyr Leu Asn
   305                 310                 315                 320
   Ser Tyr Tyr Tyr Leu Glu Ser Gln Lys Leu Ser Asp Asn Val Glu Asp
                   325                 330                 335
   Phe Thr Phe Thr Arg Ser Ile Glu Glu Ala Leu Asp Asn Ser Ala Lys
               340                 345                 350
   Val Tyr Thr Tyr Phe Pro Thr Leu Ala Asn Lys Val Asn Ala Gly Val
           355                 360                 365
   Gln Gly Gly Leu Phe Leu Met Trp Ala Asn Asp Val Val Glu Asp Phe
       370                 375                 380
   Thr Thr Asn Ile Leu Arg Lys Asp Thr Leu Asp Lys Ile Ser Asp Val
   385                 390                 395                 400
   Ser Ala Ile Ile Pro Tyr Ile Gly Pro Ala Leu Asn Ile Ser Asn Ser
                   405                 410                 415
   Val Arg Arg Gly Asn Phe Thr Glu Ala Phe Ala Val Thr Gly Val Thr
               420                 425                 430
   Ile Leu Leu Glu Ala Phe Pro Glu Phe Thr Ile Pro Ala Leu Gly Ala
   435 440 445
   Phe Val Ile Tyr Ser Lys Val Gln Glu Arg Asn Glu Ile Ile Lys Thr
   450 455 460
   Ile Asp Asn Cys Leu Glu Gln Arg Ile Lys Arg Trp Lys Asp Ser Tyr
   465 470 475 480
   Glu Trp Met Met Gly Thr Trp Leu Ser Arg Ile Ile Thr Gln Phe Asn
   485 490 495
   Asn Ile Ser Tyr Gln Met Tyr Asp Ser Leu Asn Tyr Gln Ala Gly Ala
   500 505 510
   Ile Lys Ala Lys Ile Asp Leu Glu Tyr Lys Lys Tyr Ser Gly Ser Asp
   515 520 525
   Lys Glu Asn Ile Lys Ser Gln Val Glu Asn Leu Lys Asn Ser Leu Asp
   530 535 540
   Val Lys Ile Ser Glu Ala Met Asn Asn Ile Asn Lys Phe Ile Arg Glu
   545 550 555 560
   Cys Ser Val Thr Tyr Leu Phe Lys Asn Met Leu Pro Lys Val Ile Asp
   565 570 575
   Glu Leu Asn Glu Phe Asp Arg Asn Thr Lys Ala Lys Leu Ile Asn Leu
   580 585 590
   Ile Asp Ser His Asn Ile Ile Leu Val Gly Glu Val Asp Lys Leu Lys
   595 600 605
   Ala Lys Val Asn Asn Ser Phe Gln Asn Thr Ile Pro Phe Asn Ile Phe
   610 615 620
   Ser Tyr Thr Asn Asn Ser Leu Leu Lys Asp Ile Ile Asn Glu Tyr Phe
   625 630 635 640
   Asn Asn Ile Asn Asp Ser Lys Ile Leu Ser Leu Gln Asn Arg Lys Asn
   645 650 655
   Thr Leu Val Asp Thr Ser Gly Tyr Asn Ala Glu Val Ser Glu Glu Gly
   660 665 670
   Asp Val Gln Leu Asn Pro Ile Phe Pro Phe Asp Phe Lys Leu Gly Ser
   675 680 685
   Ser Gly Glu Asp Arg Gly Lys Val Ile Val Thr Gln Asn Glu Asn Ile
   690 695 700
   Val Tyr Asn Ser Met Tyr Glu Ser Phe Ser Ile Ser Phe Trp Ile Arg
   705 710 715 720
   Ile Asn Lys Trp Val Ser Asn Leu Pro Gly Tyr Thr Ile Ile Asp Ser
   725 730 735
   Val Lys Asn Asn Ser Gly Trp Ser Ile Gly Ile Ile Ser Asn Phe Leu
   740 745 750
   Val Phe Thr Leu Lys Gln Asn Glu Asp Ser Glu Gln Ser Ile Asn Phe
   755 760 765
   Ser Tyr Asp Ile Ser Asn Asn Ala Pro Gly Tyr Asn Lys Trp Phe Phe
   770 775 780
   Val Thr Val Thr Asn Asn Met Met Gly Asn Met Lys Ile Tyr Ile Asn
   785 790 795 800
   Gly Lys Leu Ile Asp Thr Ile Lys Val Lys Glu Leu Thr Gly Ile Asn
   805 810 815
   Phe Ser Lys Thr Ile Thr Phe Glu Ile Asn Lys Ile Pro Asp Thr Gly
   820 825 830
   Leu Ile Thr Ser Asp Ser Asp Asn Ile Asn Met Trp Ile Arg Asp Phe
   835 840 845
   Tyr Ile Phe Ala Lys Glu Leu Asp Gly Lys Asp Ile Asn Ile Leu Phe
   850 855 860
   Asn Ser Leu Gln Tyr Thr Asn Val Val Lys Asp Tyr Trp Gly Asn Asp
   865 870 875 880
   Leu Arg Tyr Asn Lys Glu Tyr Tyr Met Val Asn Ile Asp Tyr Leu Asn
   885 890 895
   Arg Tyr Met Tyr Ala Asn Ser Arg Gln Ile Val Phe Asn Thr Arg Arg
   900 905 910
   Asn Asn Asn Asp Phe Asn Glu Gly Tyr Lys Ile Ile Ile Lys Arg Ile
   915 920 925
   Arg Gly Asn Thr Asn Asp Thr Arg Val Arg Gly Gly Asp Ile Leu Tyr
   930 935 940
   Phe Asp Met Thr Ile Asn Asn Lys Ala Tyr Asn Leu Phe Met Lys Asn
   945 950 955 960
   Glu Thr Met Tyr Ala Asp Asn His Ser Thr Glu Asp Ile Tyr Ala Ile
   965 970 975
   Gly Leu Arg Glu Gln Thr Lys Asp Ile Asn Asp Asn Ile Ile Phe Gln
               980                 985                 990
   Ile Gln Pro Met Asn Asn Thr Tyr  Tyr Tyr Ala Ser Gln  Ile Phe Lys
           995                 1000                 1005
   Ser Asn  Phe Asn Gly Glu Asn  Ile Ser Gly Ile Cys  Ser Ile Gly
       1010                 1015                 1020
   Thr Tyr  Arg Phe Arg Leu Gly  Gly Asp Trp Tyr Arg  His Asn Tyr
       1025                 1030                 1035
   Leu Val  Pro Thr Val Lys Gln  Gly Asn Tyr Ala Ser  Leu Leu Glu
       1040                 1045                 1050
   Ser Thr  Ser Thr His Trp Gly  Phe Val Pro Val Ser  Glu
       1055                 1060                 1065
<210> 14
   <211> 1067
   <212> PRT
   <213> Artificial Sequence
<220>
   <223> Synthetic
<400> 14
   Ala Glu Thr Lys Asn Phe Thr Asp Leu Val Glu Ala Thr Lys Trp Gly
   1 5 10 15
   Asn Ser Leu Ile Lys Ser Ala Lys Tyr Ser Ser Lys Asp Lys Met Ala
   20 25 30
   Ile Tyr Asn Tyr Thr Lys Asn Ser Ser Pro Ile Asn Thr Pro Leu Arg
   35 40 45
   Ser Ala Asn Gly Asp Val Asn Lys Leu Ser Glu Asn Ile Gln Glu Gln
   50 55 60
   Val Arg Gln Leu Asp Ser Thr Ile Ser Lys Ser Val Thr Pro Asp Ser
   65 70 75 80
   Val Tyr Val Tyr Arg Leu Leu Asn Leu Asp Tyr Leu Ser Ser Ile Thr
   85 90 95
   Gly Phe Thr Arg Glu Asp Leu His Met Leu Gln Gln Thr Asn Asn Gly
   100 105 110
   Gln Tyr Asn Glu Ala Leu Val Ser Lys Leu Asn Asn Leu Met Asn Ser
   115 120 125
   Arg Ile Tyr Arg Glu Asn Gly Tyr Ser Ser Thr Gln Leu Val Ser Gly
       130                 135                 140
   Ala Ala Leu Ala Gly Arg Pro Ile Glu Leu Lys Leu Glu Leu Pro Lys
   145                 150                 155                 160
   Gly Thr Lys Ala Ala Tyr Ile Asp Ser Lys Glu Leu Thr Ala Tyr Pro
                   165                 170                 175
   Gly Gln Gln Glu Val Leu Leu Pro Arg Gly Thr Glu Tyr Ala Val Gly
               180                 185                 190
   Ser Val Lys Leu Ser Asp Asn Lys Arg Lys Ile Ile Ile Thr Ala Val
           195                 200                 205
   Val Phe Lys Lys Cys His Lys Ala Ile Asp Gly Arg Ser Leu Tyr Asn
       210                 215                 220
   Lys Thr Leu Asp Cys Arg Glu Leu Leu Val Lys Asn Thr Asp Leu Pro
   225                 230                 235                 240
   Phe Ile Gly Asp Ile Ser Asp Val Lys Thr Asp Ile Phe Leu Arg Lys
                   245                 250                 255
   Asp Ile Asn Glu Glu Thr Glu Val Ile Tyr Tyr Pro Asp Asn Val Ser
               260                 265                 270
   Val Asp Gln Val Ile Leu Ser Lys Asn Thr Ser Glu His Gly Gln Leu
           275                 280                 285
   Asp Leu Leu Tyr Pro Ser Ile Asp Ser Glu Ser Glu Ile Leu Pro Gly
       290                 295                 300
   Glu Asn Gln Val Phe Tyr Asp Asn Arg Thr Gln Asn Val Asp Tyr Leu
   305                 310                 315                 320
   Asn Ser Tyr Tyr Tyr Leu Glu Ser Gln Lys Leu Ser Asp Asn Val Glu
                   325                 330                 335
   Asp Phe Thr Phe Thr Arg Ser Ile Glu Glu Ala Leu Asp Asn Ser Ala
               340                 345                 350
   Lys Val Tyr Thr Tyr Phe Pro Thr Leu Ala Asn Lys Val Asn Ala Gly
           355                 360                 365
   Val Gln Gly Gly Leu Phe Leu Met Trp Ala Asn Asp Val Val Glu Asp
       370                 375                 380
   Phe Thr Thr Asn Ile Leu Arg Lys Asp Thr Leu Asp Lys Ile Ser Asp
   385                 390                 395                 400
   Val Ser Ala Ile Ile Pro Tyr Ile Gly Pro Ala Leu Asn Ile Ser Asn
                   405                 410                 415
   Ser Val Arg Arg Gly Asn Phe Thr Glu Ala Phe Ala Val Thr Gly Val
   420 425 430
   Thr Ile Leu Leu Glu Ala Phe Pro Glu Phe Thr Ile Pro Ala Leu Gly
   435 440 445
   Ala Phe Val Ile Tyr Ser Lys Val Gln Glu Arg Asn Glu Ile Ile Lys
   450 455 460
   Thr Ile Asp Asn Cys Leu Glu Gln Arg Ile Lys Arg Trp Lys Asp Ser
   465 470 475 480
   Tyr Glu Trp Met Met Gly Thr Trp Leu Ser Arg Ile Ile Thr Gln Phe
   485 490 495
   Asn Asn Ile Ser Tyr Gln Met Tyr Asp Ser Leu Asn Tyr Gln Ala Gly
   500 505 510
   Ala Ile Lys Ala Lys Ile Asp Leu Glu Tyr Lys Lys Tyr Ser Gly Ser
   515 520 525
   Asp Lys Glu Asn Ile Lys Ser Gln Val Glu Asn Leu Lys Asn Ser Leu
   530 535 540
   Asp Val Lys Ile Ser Glu Ala Met Asn Asn Ile Asn Lys Phe Ile Arg
   545 550 555 560
   Glu Cys Ser Val Thr Tyr Leu Phe Lys Asn Met Leu Pro Lys Val Ile
   565 570 575
   Asp Glu Leu Asn Glu Phe Asp Arg Asn Thr Lys Ala Lys Leu Ile Asn
   580 585 590
   Leu Ile Asp Ser His Asn Ile Ile Leu Val Gly Glu Val Asp Lys Leu
   595 600 605
   Lys Ala Lys Val Asn Asn Ser Phe Gln Asn Thr Ile Pro Phe Asn Ile
   610 615 620
   Phe Ser Tyr Thr Asn Asn Ser Leu Leu Lys Asp Ile Ile Asn Glu Tyr
   625 630 635 640
   Phe Asn Asn Ile Asn Asp Ser Lys Ile Leu Ser Leu Gln Asn Arg Lys
   645 650 655
   Asn Thr Leu Val Asp Thr Ser Gly Tyr Asn Ala Glu Val Ser Glu Glu
   660 665 670
   Gly Asp Val Gln Leu Asn Pro Ile Phe Pro Phe Asp Phe Lys Leu Gly
   675 680 685
   Ser Ser Gly Glu Asp Arg Gly Lys Val Ile Val Thr Gln Asn Glu Asn
   690 695 700
   Ile Val Tyr Asn Ser Met Tyr Glu Ser Phe Ser Ile Ser Phe Trp Ile
   705 710 715 720
   Arg Ile Asn Lys Trp Val Ser Asn Leu Pro Gly Tyr Thr Ile Ile Asp
   725 730 735
   Ser Val Lys Asn Asn Ser Gly Trp Ser Ile Gly Ile Ile Ser Asn Phe
   740 745 750
   Leu Val Phe Thr Leu Lys Gln Asn Glu Asp Ser Glu Gln Ser Ile Asn
   755 760 765
   Phe Ser Tyr Asp Ile Ser Asn Asn Ala Pro Gly Tyr Asn Lys Trp Phe
   770 775 780
   Phe Val Thr Val Thr Asn Asn Met Met Gly Asn Met Lys Ile Tyr Ile
   785 790 795 800
   Asn Gly Lys Leu Ile Asp Thr Ile Lys Val Lys Glu Leu Thr Gly Ile
   805 810 815
   Asn Phe Ser Lys Thr Ile Thr Phe Glu Ile Asn Lys Ile Pro Asp Thr
   820 825 830
   Gly Leu Ile Thr Ser Asp Ser Asp Asn Ile Asn Met Trp Ile Arg Asp
   835 840 845
   Phe Tyr Ile Phe Ala Lys Glu Leu Asp Gly Lys Asp Ile Asn Ile Leu
   850 855 860
   Phe Asn Ser Leu Gln Tyr Thr Asn Val Val Lys Asp Tyr Trp Gly Asn
   865 870 875 880
   Asp Leu Arg Tyr Asn Lys Glu Tyr Tyr Met Val Asn Ile Asp Tyr Leu
   885 890 895
   Asn Arg Tyr Met Tyr Ala Asn Ser Arg Gln Ile Val Phe Asn Thr Arg
   900 905 910
   Arg Asn Asn Asn Asp Phe Asn Glu Gly Tyr Lys Ile Ile Ile Lys Arg
   915 920 925
   Ile Arg Gly Asn Thr Asn Asp Thr Arg Val Arg Gly Gly Asp Ile Leu
   930 935 940
   Tyr Phe Asp Met Thr Ile Asn Asn Lys Ala Tyr Asn Leu Phe Met Lys
   945 950 955 960
   Asn Glu Thr Met Tyr Ala Asp Asn His Ser Thr Glu Asp Ile Tyr Ala
   965 970 975
   Ile Gly Leu Arg Glu Gln Thr Lys Asp Ile Asn Asp Asn Ile Ile Phe
   980 985 990
   Gln Ile Gln Pro Met Asn Asn Thr Tyr Tyr Tyr Ala Ser Gln Ile Phe
   995 1000 1005
   Lys Ser Asn Phe Asn Gly Glu Asn Ile Ser Gly Ile Cys Ser Ile
   1010 1015 1020
   Gly Thr Tyr Arg Phe Arg Leu Gly Gly Asp Trp Tyr Arg His Asn
   1025 1030 1035
   Tyr Leu Val Pro Thr Val Lys Gln Gly Asn Tyr Ala Ser Leu Leu
   1040 1045 1050
   Glu Ser Thr Ser Thr His Trp Gly Phe Val Pro Val Ser Glu
   1055 1060 1065
<210> 15
   <211> 682
   <212> PRT
   <213> Artificial Sequence
<220>
   <223> Synthetic
<400> 15
   Ala Tyr Ser Asn Thr Tyr Gln Glu Phe Thr Asn Ile Asp Gln Ala Lys
   1               5                   10                  15
   Ala Trp Gly Asn Ala Gln Tyr Lys Lys Tyr Gly Leu Ser Lys Ser Glu
               20                  25                  30
   Lys Glu Ala Ile Val Ser Tyr Thr Lys Ser Ala Ser Glu Ile Asn Gly
           35                  40                  45
   Lys Leu Arg Gln Asn Lys Gly Val Ile Asn Gly Phe Pro Ser Asn Leu
       50                  55                  60
   Ile Lys Gln Val Glu Leu Leu Asp Lys Ser Phe Asn Lys Met Lys Thr
   65                  70                  75                  80
   Pro Glu Asn Ile Met Leu Phe Arg Gly Asp Asp Pro Ala Tyr Leu Gly
                   85                  90                  95
   Thr Glu Phe Gln Asn Thr Leu Leu Asn Ser Asn Gly Thr Ile Asn Lys
               100                 105                 110
   Thr Ala Phe Glu Lys Ala Lys Ala Lys Phe Leu Asn Lys Asp Arg Leu
           115                 120                 125
   Glu Tyr Gly Tyr Ile Ser Thr Ser Leu Met Asn Val Ser Gln Phe Ala
       130                 135                 140
   Gly Arg Pro Ile Ile Thr Lys Phe Lys Val Ala Lys Gly Ser Lys Ala
   145                 150                 155                 160
   Gly Tyr Ile Asp Pro Ile Ser Ala Phe Ala Gly Gln Leu Glu Met Leu
                   165                 170                 175
   Leu Pro Arg His Ser Thr Tyr His Ile Asp Asp Met Arg Leu Ser Ser
               180                 185                 190
   Asp Gly Lys Gln Ile Ile Ile Thr Ala Thr Met Met Gly Thr Ala Ile
           195                 200                 205
   Asn Pro Lys Cys His Lys Ala Ile Asp Gly Arg Ser Leu Tyr Asn Lys
       210                 215                 220
   Thr Leu Asp Cys Gly Ser Phe Gln Asn Thr Ile Pro Phe Asn Ile Phe
   225                 230                 235                 240
   Ser Tyr Thr Asn Asn Ser Leu Leu Lys Asp Ile Ile Asn Glu Tyr Phe
                   245                 250                 255
   Asn Asn Ile Asn Asp Ser Lys Ile Leu Ser Leu Gln Asn Arg Lys Asn
               260                 265                 270
   Thr Leu Val Asp Thr Ser Gly Tyr Asn Ala Glu Val Ser Glu Glu Gly
           275                 280                 285
   Asp Val Gln Leu Asn Pro Ile Phe Pro Phe Asp Phe Lys Leu Gly Ser
       290                 295                 300
   Ser Gly Glu Asp Arg Gly Lys Val Ile Val Thr Gln Asn Glu Asn Ile
   305                 310                 315                 320
   Val Tyr Asn Ser Met Tyr Glu Ser Phe Ser Ile Ser Phe Trp Ile Arg
                   325                 330                 335
   Ile Asn Lys Trp Val Ser Asn Leu Pro Gly Tyr Thr Ile Ile Asp Ser
               340                 345                 350
   Val Lys Asn Asn Ser Gly Trp Ser Ile Gly Ile Ile Ser Asn Phe Leu
           355                 360                 365
   Val Phe Thr Leu Lys Gln Asn Glu Asp Ser Glu Gln Ser Ile Asn Phe
       370                 375                 380
   Ser Tyr Asp Ile Ser Asn Asn Ala Pro Gly Tyr Asn Lys Trp Phe Phe
   385                 390                 395                 400
   Val Thr Val Thr Asn Asn Met Met Gly Asn Met Lys Ile Tyr Ile Asn
   405 410 415
   Gly Lys Leu Ile Asp Thr Ile Lys Val Lys Glu Leu Thr Gly Ile Asn
   420 425 430
   Phe Ser Lys Thr Ile Thr Phe Glu Ile Asn Lys Ile Pro Asp Thr Gly
   435 440 445
   Leu Ile Thr Ser Asp Ser Asp Asn Ile Asn Met Trp Ile Arg Asp Phe
   450 455 460
   Tyr Ile Phe Ala Lys Glu Leu Asp Gly Lys Asp Ile Asn Ile Leu Phe
   465 470 475 480
   Asn Ser Leu Gln Tyr Thr Asn Val Val Lys Asp Tyr Trp Gly Asn Asp
   485 490 495
   Leu Arg Tyr Asn Lys Glu Tyr Tyr Met Val Asn Ile Asp Tyr Leu Asn
   500 505 510
   Arg Tyr Met Tyr Ala Asn Ser Arg Gln Ile Val Phe Asn Thr Arg Arg
   515 520 525
   Asn Asn Asn Asp Phe Asn Glu Gly Tyr Lys Ile Ile Ile Lys Arg Ile
   530 535 540
   Arg Gly Asn Thr Asn Asp Thr Arg Val Arg Gly Gly Asp Ile Leu Tyr
   545 550 555 560
   Phe Asp Met Thr Ile Asn Asn Lys Ala Tyr Asn Leu Phe Met Lys Asn
   565 570 575
   Glu Thr Met Tyr Ala Asp Asn His Ser Thr Glu Asp Ile Tyr Ala Ile
   580 585 590
   Gly Leu Arg Glu Gln Thr Lys Asp Ile Asn Asp Asn Ile Ile Phe Gln
   595 600 605
   Ile Gln Pro Met Asn Asn Thr Tyr Tyr Tyr Ala Ser Gln Ile Phe Lys
   610 615 620
   Ser Asn Phe Asn Gly Glu Asn Ile Ser Gly Ile Cys Ser Ile Gly Thr
   625 630 635 640
   Tyr Arg Phe Arg Leu Gly Gly Asp Trp Tyr Arg His Asn Tyr Leu Val
   645 650 655
   Pro Thr Val Lys Gln Gly Asn Tyr Ala Ser Leu Leu Glu Ser Thr Ser
   660 665 670
   Thr His Trp Gly Phe Val Pro Val Ser Glu
   675 680
<210> 16
   <211> 12
   <212> PRT
   <213> Artificial Sequence
<220>
   <223> Synthetic
<400> 16
   Cys Ser Ala Ile Glu Gly Arg Ala Pro Gly Ile Cys
   1 5 10
<210> 17
   <211> 20
   <212> PRT
   <213> Artificial Sequence
<220>
   <223> Synthetic
<400> 17
   Cys Gly Ile Glu Gly Arg Ala Pro Gly Pro Gly Ser Ser Val Gly Ser
   1 5 10 15
   Ser Leu Ser Cys
   20
<210> 18
   <211> 11
   <212> PRT
   <213> Artificial Sequence
<220>
   <223> Synthetic
<400> 18
   Cys Gly Leu Val Pro Arg Gly Ser Gly Pro Cys
   1 5 10
<210> 19
   <211> 20
   <212> PRT
   <213> Artificial Sequence
<220>
   <223> Synthetic
<400> 19
   Cys Gly Leu Val Pro Arg Gly Ser Gly Pro Gly Ser Ser Val Gly Ser
   1 5 10 15
   Ser Leu Ser Cys
   20
<210> 20
   <211> 13
   <212> PRT
   <213> Artificial Sequence
<220>
   <223> Synthetic
<400> 20
   Cys Lys Ser Asp Asp Asp Asp Lys Ala Pro Gly Ile Cys
   1 5 10
<210> 21
   <211> 22
   <212> PRT
   <213> Artificial Sequence
<220>
   <223> Synthetic
<400> 21
   Cys Lys Ser Glu Glu Lys Leu Tyr Asp Asp Asp Asp Lys Asp Arg Trp
   1 5 10 15
   Gly Ser Ser Arg Ile Cys
   20
<210> 22
   <211> 17
   <212> PRT
   <213> Clostridium Botulinum
<400> 22
   Cys His Lys Ala Ile Asp Gly Arg Ser Leu Tyr Asn Lys Thr Leu Asp
   1 5 10 15
   Cys
<210> 23
   <211> 10
   <212> PRT
   <213> Artificial Sequence
<220>
   <223> Synthetic
<400> 23
   Cys Gly Leu Val Pro Ala Gly Ser Gly Pro
   1 5 10
<210> 24
   <211> 17
   <212> PRT
   <213> Artificial Sequence
<220>
   <223> Synthetic
<400> 24
   Cys Gly Leu Val Pro Ala Gly Ser Gly Pro Ser Ala Gly Ser Ser Ala
   1 5 10 15
   Cys